# EUROPEAN PATENT APPLICATION

(11) **EP 4 471 149 A2**
(43) Date of publication of application: **04.12.2024**
(21) Application number: 24175045.4
(22) Date of filing: 30.10.2020
(51) Int. Cl.: C12N 15/82

(54) **TYPE V CRISPR-CAS BASE EDITORS AND METHODS OF USE THEREOF**

(30) Priority: 30.10.2019 US 201962927914 P
(62) Divisional of application: 20880833.7
(71) Applicant: Pairwise Plants Services, Inc., Durham, NC 27701 (US)
(72) Inventor: KIM, Yongjoo, Durham, North Carolina 27701 (US)
(74) Representative: Algemeen Octrooi- en Merkenbureau B.V.

(57) **Abstract**

This invention relates to Type V CRISPR-Cas effector proteins, deaminases, and fusion and recruiting nucleic acid constructs thereof. The invention further relates methods of targeted nucleic acid modification utilizing the same.

## Description

### STATEMENT REGARDING ELECTRONIC FILING OF A SEQUENCE

### LISTING

A Sequence Listing in ASCII text format, submitted under 37 C.F.R. § 1.821, entitled 1499-10WO_ST25.txt, 351,999 bytes in size, generated on October 30, 2020 and filed via EFS-Web, is provided in lieu of a paper copy. This Sequence Listing is hereby incorporated herein by reference into the specification for its disclosures.

### FIELD OF THE INVENTION

This invention relates to Type V CRISPR-Cas effector proteins, deaminases, and fusion and recruiting nucleic acid constructs thereof. The invention further relates methods of targeted nucleic acid modification utilizing the same.

### BACKGROUND OF THE INVENTION

Gene editing is the process of utilizing a site-directed nuclease to introduce variation at targeted genomic locations. C to T base editing may be achieved with base editors that use Cas9 as the targeting module. For example, Komor et al. (Sci Advances 3(8):eaao4774) (2017)) and Koblan et al. (NatBiotechnol 36(9):843-846 (2018) disclose base editors that use Cas9 as a fusion protein including APOBEC1 deaminase, Cas9 nickase (D10A), and 2 copies of Uracil glycosylase inhibitor (UGI). Li et al. (Nat Biotechnol. 36(4):324-327 (2018)) replaces Cas9 with deactivated Cpfl. Although active in human cells, this Cpfl construct is less efficient than its Cas9 counterparts. To make base editing more useful across a greater number of organisms, including plants, new base editing tools are needed.

### SUMMARY OF THE INVENTION

One aspect of the invention provides a method of modifying a target nucleic acid, the method comprising contacting the target nucleic acid with: (a) a Type V CRISPR-Cas effector protein; (b) a deaminase, optionally wherein the target nucleic acid is contacted with two or more deaminase; and(c) a guide nucleic acid, wherein the deaminase is recruited to the Type V CRISPR-Cas effector protein (e.g., recruited via a protein to protein interaction, RNA to protein interaction, and/or chemical interaction), thereby modifying the target nucleic acid, optionally wherein the Type V CRISPR-Cas effector protein, the deaminase and guide nucleic acid are co-expressed.

A second aspect of the invention provides a method of modifying a target nucleic acid, the method comprising contacting the target nucleic acid with: (a) a Type V CRISPR-Cas fusion protein comprising a Type V CRISPR-Cas effector protein fused to a peptide tag (e.g., an epitope or a multimerized epitope); (b) a deaminase fusion protein comprising a deaminase fused to an affinity polypeptide that binds to the peptide tag, optionally wherein the target nucleic acid is contacted with two or more deaminase fusion proteins; and (c) a guide nucleic acid, optionally wherein the Type V CRISPR-Cas fusion protein, the deaminase fusion protein and guide nucleic acid are co-expressed, thereby modifying the target nucleic acid.

A third aspect of the invention provides a method of modifying a target nucleic acid, the method comprising contacting the target nucleic acid with: (a) a Type V CRISPR-Cas fusion protein comprising a Type V CRISPR-Cas effector protein fused to an affinity polypeptide that binds to a peptide tag; (b) a deaminase fusion protein comprising a deaminase fused to the peptide tag (e.g., an epitope or a multimerized epitope), optionally wherein the target nucleic acid is contacted with two or more deaminase fusion proteins; and (c) a guide nucleic acid, optionally wherein the Type V CRISPR-Cas fusion protein, the deaminase fusion protein and guide nucleic acid are co-expressed, thereby modifying the target nucleic acid.

A fourth aspect provides a method of modifying a target nucleic acid, the method comprising contacting the target nucleic acid with: (a) a Type V CRISPR-Cas effector protein; (b) a recruiting guide nucleic acid comprising a guide nucleic acid linked to an RNA recruiting motif, and (c) a deaminase fusion protein comprising a deaminase fused to an affinity polypeptide that binds to the RNA recruiting motif, optionally wherein the target nucleic acid is contacted with two or more deaminase fusion proteins; and optionally wherein the Type V CRISPR-Cas effector protein, the deaminase fusion protein and the recruiting guide nucleic acid are co-expressed, thereby modifying the target nucleic acid.

A fifth aspect of the invention provides a nucleic acid construct comprising: (a) a Type V CRISPR-Cas fusion protein comprising a Type V CRISPR-Cas effector protein fused to a peptide tag; (b) a deaminase fusion protein comprising a deaminase fused to an affinity polypeptide that binds to the peptide tag; and (c) a guide nucleic acid.

A sixth aspect of the invention provides a nucleic acid construct comprising: (a) a Type V CRISPR-Cas effector protein; (b) a recruiting guide nucleic acid comprising a guide nucleic acid linked to an RNA recruiting motif; and (c) a deaminase fusion protein comprising a deaminase fused to an affinity polypeptide that binds to the RNA recruiting motif.

A seventh aspect of the invention provides a Type V Clustered Regularly Interspaced Short Palindromic Repeats (CRISPR)-associated (Cas) (CRISPR-Cas) system comprising: (a) a Type V CRISPR-Cas fusion protein comprising a Type V CRISPR-Cas effector protein fused to a peptide tag; (b) a deaminase fusion protein comprising a deaminase fused to an affinity polypeptide that binds to the peptide tag; and (c) a guide nucleic acid comprising a spacer sequence and a repeat sequence, wherein the guide nucleic acid is capable of forming a complex with the Type V CRISPR-Cas effector protein of the Type V CRISPR-Cas fusion protein and the spacer sequence is capable of hybridizing to a target nucleic acid, thereby guiding the Type V CRISPR-Cas fusion protein to the target nucleic acid, and wherein the deaminase fusion protein is recruited to the Type V CRISPR-Cas fusion protein and target nucleic acid by the binding of the affinity polypeptide to the peptide tag that is fused to the Type V CRISPR-Cas fusion protein, whereby the system is capable of modifying (e.g., cleaving or editing) the target nucleic acid.

A eighth aspect of the invention provides a Type V Clustered Regularly Interspaced Short Palindromic Repeats (CRISPR)-associated (Cas) (CRISPR-Cas) system comprising: (a) a Type V CRISPR-Cas effector protein; (b) a recruiting guide nucleic acid comprising a guide nucleic acid linked to an RNA recruiting motif, and (c) a deaminase fusion protein comprising a deaminase fused to an affinity polypeptide that binds to the RNA recruiting motif, wherein the recruiting guide nucleic acid comprises a spacer sequence and a repeat sequence, wherein the guide nucleic acid is capable of forming a complex with the Type V CRISPR-Cas effector protein and the recruiting guide nucleic acid is capable of hybridizing to a target nucleic acid, thereby guiding the Type V CRISPR-Cas effector protein to the target nucleic acid, and wherein the deaminase fusion protein is recruited to the Type V CRISPR-Cas effector protein and target nucleic acid by the binding of the affinity polypeptide to the RNA recruiting motif that is fused to the recruiting guide nucleic acid, whereby the system is capable of modifying (e.g., cleaving or editing) the target nucleic acid.

The invention further provides expression cassettes and/or vectors comprising the nucleic acid constructs of the invention, and cells comprising the polypeptides, fusion proteins and/or nucleic acid constructs of the invention. Additionally, the invention provides kits comprising the nucleic acid constructs of the invention and expression cassettes, vectors and/or cells comprising the same.

It is noted that aspects of the invention described with respect to one embodiment, may be incorporated in a different embodiment although not specifically described relative thereto. That is, all embodiments and/or features of any embodiment can be combined in any way and/or combination. Applicant reserves the right to change any originally filed claim and/or file any new claim accordingly, including the right to be able to amend any originally filed claim to depend from and/or incorporate any feature of any other claim or claims although not originally claimed in that manner. These and other objects and/or aspects of the present invention are explained in detail in the specification set forth below. Further features, advantages and details of the present invention will be appreciated by those of ordinary skill in the art from a reading of the figures and the detailed description of the preferred embodiments that follow, such description being merely illustrative of the present invention.

### BRIEF DESCRIPTION OF THE DRAWINGS

**Fig. 1** is a graph showing C to T editing efficiencies for editing systems using pWg120029 as the guide nucleic acid according to some embodiments of the present invention.
**Fig. 2** is a graph showing C to T editing efficiencies for editing systems using pWg120360 as the guide nucleic acid according to some embodiments of the present invention.
**Fig. 3** is a graph showing C to T editing efficiencies for editing systems using pWg120300 as the guide nucleic acid according to some embodiments of the present invention.
**Fig. 4** is a graph showing C to T editing efficiencies for editing systems using pWg120301 as the guide nucleic acid according to some embodiments of the present invention.
**Fig. 5** is a graph showing A to G editing efficiencies for editing systems using different the guide nucleic acids according to some embodiments of the present invention.

### DETAILED DESCRIPTION

The present invention now will be described hereinafter with reference to the accompanying drawings and examples, in which embodiments of the invention are shown. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. Thus, the invention contemplates that in some embodiments of the invention, any feature or combination of features set forth herein can be excluded or omitted. In addition, numerous variations and additions to the various embodiments suggested herein will be apparent to those skilled in the art in light of the instant disclosure, which do not depart from the instant invention. Hence, the following descriptions are intended to illustrate some particular embodiments of the invention, and not to exhaustively specify all permutations, combinations and variations thereof.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

All publications, patent applications, patents and other references cited herein are incorporated by reference in their entireties for the teachings relevant to the sentence and/or paragraph in which the reference is presented.

Unless the context indicates otherwise, it is specifically intended that the various features of the invention described herein can be used in any combination. Moreover, the present invention also contemplates that in some embodiments of the invention, any feature or combination of features set forth herein can be excluded or omitted. To illustrate, if the specification states that a composition comprises components A, B and C, it is specifically intended that any of A, B or C, or a combination thereof, can be omitted and disclaimed singularly or in any combination.

As used in the description of the invention and the appended claims, the singular forms "a," "an" and "the" are intended to include the plural forms as well, unless the context clearly indicates otherwise.

Also as used herein, "and/or" refers to and encompasses any and all possible combinations of one or more of the associated listed items, as well as the lack of combinations when interpreted in the alternative ("or").

The term "about," as used herein when referring to a measurable value such as an amount or concentration and the like, is meant to encompass variations of ± 10%, ± 5%, ± 1%, ± 0.5%, or even ± 0.1% of the specified value as well as the specified value. For example, "about X" where X is the measurable value, is meant to include X as well as variations of ± 10%, ± 5%, ± 1%, ± 0.5%, or even ± 0.1% of X. A range provided herein for a measureable value may include any other range and/or individual value therein.

As used herein, phrases such as "between X and Y" and "between about X and Y" should be interpreted to include X and Y. As used herein, phrases such as "between about X and Y" mean "between about X and about Y" and phrases such as "from about X to Y" mean "from about X to about Y."

Recitation of ranges of values herein are merely intended to serve as a shorthand method of referring individually to each separate value falling within the range, unless otherwise indicated herein, and each separate value is incorporated into the specification as if it were individually recited herein. For example, if the range 10 to 15 is disclosed, then 11, 12, 13, and 14 are also disclosed.

The term "comprise," "comprises" and "comprising" as used herein, specify the presence of the stated features, integers, steps, operations, elements, and/or components, but do not preclude the presence or addition of one or more other features, integers, steps, operations, elements, components, and/or groups thereof.

As used herein, the transitional phrase "consisting essentially of" means that the scope of a claim is to be interpreted to encompass the specified materials or steps recited in the claim and those that do not materially affect the basic and novel characteristic(s) of the claimed invention. Thus, the term "consisting essentially of" when used in a claim of this invention is not intended to be interpreted to be equivalent to "comprising."

As used herein, the terms "increase," "increasing," "enhance," "enhancing," "improve" and "improving" (and grammatical variations thereof) describe an elevation of at least about 5%, 10%, 15%, 20%, 25%, 30%, 35%, 40%, 45%, 50%, 55%, 60%, 65%, 70%, 75%, 80%, 85%, 90%, 95%, 100%, 150%, 200%, 300%, 400%, 500% or more as compared to a control.

As used herein, the terms "reduce," "reduced," "reducing," "reduction," "diminish," and "decrease" (and grammatical variations thereof), describe, for example, a decrease of at least about 5%, 10%, 15%, 20%, 25%, 30%, 35%, 40%, 45%, 50%, 55%, 60%, 65%, 70%, 75%, 80%, 85%, 90%, 95%, 97%, 98%, 99%, or 100% as compared to a control. In particular embodiments, the reduction can result in no or essentially no (*i.e.*, an insignificant amount, e.g., less than about 10% or even 5%) detectable activity or amount.

A "heterologous" or a "recombinant" nucleotide sequence is a nucleotide sequence not naturally associated with a host cell into which it is introduced, including non- naturally occurring multiple copies of a naturally occurring nucleotide sequence.

A "native" or "wild type" nucleic acid, nucleotide sequence, polypeptide or amino acid sequence refers to a naturally occurring or endogenous nucleic acid, nucleotide sequence, polypeptide or amino acid sequence. Thus, for example, a "wild type mRNA" is an mRNA that is naturally occurring in or endogenous to the reference organism. A "homologous" nucleic acid sequence is a nucleotide sequence naturally associated with a host cell into which it is introduced.

As used herein, the terms "nucleic acid," "nucleic acid molecule," "nucleotide sequence" and "polynucleotide" refer to RNA or DNA that is linear or branched, single or double stranded, or a hybrid thereof. The term also encompasses RNA/DNA hybrids. When dsRNA is produced synthetically, less common bases, such as inosine, 5-methylcytosine, 6-methyladenine, hypoxanthine and others can also be used for antisense, dsRNA, and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, or the 2'-hydroxy in the ribose sugar group of the RNA can also be made.

As used herein, the term "nucleotide sequence" refers to a heteropolymer of nucleotides or the sequence of these nucleotides from the 5' to 3' end of a nucleic acid molecule and includes DNA or RNA molecules, including cDNA, a DNA fragment or portion, genomic DNA, synthetic (e.g., chemically synthesized) DNA, plasmid DNA, mRNA, and anti-sense RNA, any of which can be single stranded or double stranded. The terms "nucleotide sequence" "nucleic acid," "nucleic acid molecule," "nucleic acid construct," "recombinant nucleic acid," "oligonucleotide" and "polynucleotide" are also used interchangeably herein to refer to a heteropolymer of nucleotides. Nucleic acid molecules and/or nucleotide sequences provided herein are presented herein in the 5' to 3' direction, from left to right and are represented using the standard code for representing the nucleotide characters as set forth in the U.S. sequence rules, 37 CFR §§1.821 - 1.825 and the World Intellectual Property Organization (WIPO) Standard ST.25. A "5' region" as used herein can mean the region of a polynucleotide that is nearest the 5' end of the polynucleotide. Thus, for example, an element in the 5' region of a polynucleotide can be located anywhere from the first nucleotide located at the 5' end of the polynucleotide to the nucleotide located halfway through the polynucleotide. A "3' region" as used herein can mean the region of a polynucleotide that is nearest the 3' end of the polynucleotide. Thus, for example, an element in the 3' region of a polynucleotide can be located anywhere from the first nucleotide located at the 3' end of the polynucleotide to the nucleotide located halfway through the polynucleotide.

As used herein, the term "gene" refers to a nucleic acid molecule capable of being used to produce mRNA, antisense RNA, miRNA, anti-microRNA antisense oligodeoxyribonucleotide (AMO) and the like. Genes may or may not be capable of being used to produce a functional protein or gene product. Genes can include both coding and non-coding regions (e.g., introns, regulatory elements, promoters, enhancers, termination sequences and/or 5' and 3' untranslated regions). A gene may be "isolated" by which is meant a nucleic acid that is substantially or essentially free from components normally found in association with the nucleic acid in its natural state. Such components include other cellular material, culture medium from recombinant production, and/or various chemicals used in chemically synthesizing the nucleic acid.

The term "mutation" refers to point mutations (e.g., missense, or nonsense, or insertions or deletions of single base pairs that result in frame shifts), insertions, deletions, and/or truncations. When the mutation is a substitution of a residue within an amino acid sequence with another residue, or a deletion or insertion of one or more residues within a sequence, the mutations are typically described by identifying the original residue followed by the position of the residue within the sequence and by the identity of the newly substituted residue.

The terms "complementary" or "complementarity," as used herein, refer to the natural binding of polynucleotides under permissive salt and temperature conditions by base-pairing. For example, the sequence "A-G-T" (5' to 3') binds to the complementary sequence "T-C-A" (3' to 5'). Complementarity between two single-stranded molecules may be "partial," in which only some of the nucleotides bind, or it may be complete when total complementarity exists between the single stranded molecules. The degree of complementarity between nucleic acid strands has significant effects on the efficiency and strength of hybridization between nucleic acid strands.

"Complement" as used herein can mean 100% complementarity with the comparator nucleotide sequence or it can mean less than 100% complementarity (e.g., "substantially complementary," e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, and the like, complementarity).

A "portion" or "fragment" of a nucleotide sequence or polypeptide will be understood to mean a nucleotide sequence or polypeptide of reduced length (e.g., reduced by 1, 2, 3, 4, 5, 6, 7, 8, 9, 10, 11, 12, 13, 14, 15, 16, 17, 18, 19, 20 or more residue(s) (e.g., nucleotide(s) or peptide(s)) relative to a reference nucleotide sequence or polypeptide, respectively, and comprising, consisting essentially of and/or consisting of contiguous residues identical or almost identical (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identical) to the reference nucleotide sequence or polypeptide. Such a nucleic acid fragment or portion according to the invention may be, where appropriate, included in a larger polynucleotide of which it is a constituent. As an example, a repeat sequence of guide nucleic acid of this invention may comprise a portion of a wild type CRISPR-Cas repeat sequence (e.g., a wild type Type V CRISPR Cas repeat, e.g., a repeat from the CRISPR Cas system that includes, but is not limited to, Cas12a (Cpfl), Cas12b, Cas12c (C2c3), Cas12d (CasY), Cas12e (CasX), Cas12g, Cas12h, Cas12i, C2c1, C2c4, C2c5, C2c8, C2c9, C2c10, Cas14a, Cas14b, and/or Cas14c, and the like).

Different nucleic acids or proteins having homology are referred to herein as "homologues." The term homologue includes homologous sequences from the same and other species and orthologous sequences from the same and other species. "Homology" refers to the level of similarity between two or more nucleic acid and/or amino acid sequences in terms of percent of positional identity (i.e., sequence similarity or identity). Homology also refers to the concept of similar functional properties among different nucleic acids or proteins. Thus, the compositions and methods of the invention further comprise homologues to the nucleotide sequences and polypeptide sequences of this invention. "Orthologous," as used herein, refers to homologous nucleotide sequences and/ or amino acid sequences in different species that arose from a common ancestral gene during speciation. A homologue of a nucleotide sequence of this invention has a substantial sequence identity (e.g., at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or 100%) to said nucleotide sequence of the invention.

As used herein "sequence identity" refers to the extent to which two optimally aligned polynucleotide or polypeptide sequences are invariant throughout a window of alignment of components, e.g., nucleotides or amino acids. "Identity" can be readily calculated by known methods including, but not limited to, those described in: Computational Molecular Biology (Lesk, A. M., ed.) Oxford University Press, New York (1988); Biocomputing: Informatics and Genome Projects (Smith, D. W., ed.) Academic Press, New York (1993); Computer Analysis of Sequence Data, Part I (Griffin, A. M., and Griffin, H. G., eds.) Humana Press, New Jersey (1994); Sequence Analysis in Molecular Biology (von Heinje, G., ed.) Academic Press (1987); and Sequence Analysis Primer (Gribskov, M. and Devereux, J., eds.) Stockton Press, New York (1991).

As used herein, the term "percent sequence identity" or "percent identity" refers to the percentage of identical nucleotides in a linear polynucleotide sequence of a reference ("query") polynucleotide molecule (or its complementary strand) as compared to a test ("subject") polynucleotide molecule (or its complementary strand) when the two sequences are optimally aligned. In some embodiments, "percent identity" can refer to the percentage of identical amino acids in an amino acid sequence as compared to a reference polypeptide.

As used herein, the phrase "substantially identical," or "substantial identity" in the context of two nucleic acid molecules, nucleotide sequences or protein sequences, refers to two or more sequences or subsequences that have at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or 100% nucleotide or amino acid residue identity, when compared and aligned for maximum correspondence, as measured using one of the following sequence comparison algorithms or by visual inspection. In some embodiments of the invention, the substantial identity exists over a region of consecutive nucleotides of a nucleotide sequence of the invention that is about 10 nucleotides to about 20 nucleotides, about 10 nucleotides to about 25 nucleotides, about 10 nucleotides to about 30 nucleotides, about 15 nucleotides to about 25 nucleotides, about 30 nucleotides to about 40 nucleotides, about 50 nucleotides to about 60 nucleotides, about 70 nucleotides to about 80 nucleotides, about 90 nucleotides to about 100 nucleotides, or more nucleotides in length, and any range therein, up to the full length of the sequence. In some embodiments, the nucleotide sequences can be substantially identical over at least about 20 nucleotides (e.g., about 20, 21, 22, 23, 24, 25, 26, 27, 28, 29, 30, 31, 32, 33, 34, 35, 36, 37, 38, 39, 40 nucleotides). In some embodiments, a substantially identical nucleotide or protein sequence performs substantially the same function as the nucleotide (or encoded protein sequence) to which it is substantially identical.

For sequence comparison, typically one sequence acts as a reference sequence to which test sequences are compared. When using a sequence comparison algorithm, test and reference sequences are entered into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

Optimal alignment of sequences for aligning a comparison window are well known to those skilled in the art and may be conducted by tools such as the local homology algorithm of Smith and Waterman, the homology alignment algorithm of Needleman and Wunsch, the search for similarity method of Pearson and Lipman, and optionally by computerized implementations of these algorithms such as GAP, BESTFIT, FASTA, and TFASTA available as part of the GCG^{®} Wisconsin Package^{®} (Accelrys Inc., San Diego, CA). An "identity fraction" for aligned segments of a test sequence and a reference sequence is the number of identical components which are shared by the two aligned sequences divided by the total number of components in the reference sequence segment, e.g., the entire reference sequence or a smaller defined part of the reference sequence. Percent sequence identity is represented as the identity fraction multiplied by 100. The comparison of one or more polynucleotide sequences may be to a full-length polynucleotide sequence or a portion thereof, or to a longer polynucleotide sequence. For purposes of this invention "percent identity" may also be determined using BLASTX version 2.0 for translated nucleotide sequences and BLASTN version 2.0 for polynucleotide sequences.

Two nucleotide sequences may also be considered substantially complementary when the two sequences hybridize to each other under stringent conditions. In some representative embodiments, two nucleotide sequences considered to be substantially complementary hybridize to each other under highly stringent conditions.

"Stringent hybridization conditions" and "stringent hybridization wash conditions" in the context of nucleic acid hybridization experiments such as Southern and Northern hybridizations are sequence dependent, and are different under different environmental parameters. An extensive guide to the hybridization of nucleic acids is found in Tij ssen Laboratory Techniques in Biochemistry and Molecular Biology-Hybridization with Nucleic Acid Probes part I chapter 2 "Overview of principles of hybridization and the strategy of nucleic acid probe assays" Elsevier, New York (1993). Generally, highly stringent hybridization and wash conditions are selected to be about 5°C lower than the thermal melting point (Tₘ) for the specific sequence at a defined ionic strength and pH.

The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleotide sequences which have more than 100 complementary residues on a filter in a Southern or northern blot is 50% formamide with 1 mg of heparin at 42°C, with the hybridization being carried out overnight. An example of highly stringent wash conditions is 0.1 5M NaCl at 72°C for about 15 minutes. An example of stringent wash conditions is a 0.2x SSC wash at 65°C for 15 minutes (*see*, Sambrook, *infra*, for a description of SSC buffer). Often, a high stringency wash is preceded by a low stringency wash to remove background probe signal. An example of a medium stringency wash for a duplex of, e.g., more than 100 nucleotides, is 1x SSC at 45°C for 15 minutes. An example of a low stringency wash for a duplex of, *e.g.*, more than 100 nucleotides, is 4-6x SSC at 40°C for 15 minutes. For short probes (e.g., about 10 to 50 nucleotides), stringent conditions typically involve salt concentrations of less than about 1.0 M Na ion, typically about 0.01 to 1.0 M Na ion concentration (or other salts) at pH 7.0 to 8.3, and the temperature is typically at least about 30°C. Stringent conditions can also be achieved with the addition of destabilizing agents such as formamide. In general, a signal to noise ratio of 2x (or higher) than that observed for an unrelated probe in the particular hybridization assay indicates detection of a specific hybridization. Nucleotide sequences that do not hybridize to each other under stringent conditions are still substantially identical if the proteins that they encode are substantially identical. This can occur, for example, when a copy of a nucleotide sequence is created using the maximum codon degeneracy permitted by the genetic code.

A polynucleotide and/or recombinant nucleic acid construct of this invention can be codon optimized for expression. In some embodiments, a polynucleotide, nucleic acid construct, expression cassette, and/or vector of the invention (comprising/encoding a Type V CRISPR-Cas effector protein, a deaminase fusion protein, and a recruiting guide nucleic acid or a Type V CRISPR-Cas fusion protein, a deaminase fusion protein, and a guide nucleic acid) may be codon optimized for expression in an organism (e.g., an animal, a plant, a fungus, an archaeon, or a bacterium). In some embodiments, the codon optimized nucleic acid constructs, polynucleotides, expression cassettes, and/or vectors of the invention have about 70% to about 99.9% (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%. 99.9% or 100%) identity or more to the reference nucleic acid constructs, polynucleotides, expression cassettes, and/or vectors but which have not been codon optimized.

In any of the embodiments described herein, a polynucleotide or nucleic acid construct of the invention may be operatively associated with a variety of promoters and/or other regulatory elements for expression in a an organism or cell thereof (e.g., a plant and/or a cell of a plant). Thus, in some embodiments, a polynucleotide or nucleic acid construct of this invention may further comprise one or more promoters, introns, enhancers, and/or terminators operably linked to one or more nucleotide sequences. In some embodiments, a promoter may be operably associated with an intron (e.g., Ubi1 promoter and intron). In some embodiments, a promoter associated with an intron maybe referred to as a "promoter region" (e.g., Ubi1 promoter and intron).

By "operably linked" or "operably associated" as used herein in reference to polynucleotides, it is meant that the indicated elements are functionally related to each other, and are also generally physically related. Thus, the term "operably linked" or "operably associated" as used herein, refers to nucleotide sequences on a single nucleic acid molecule that are functionally associated. Thus, a first nucleotide sequence that is operably linked to a second nucleotide sequence means a situation when the first nucleotide sequence is placed in a functional relationship with the second nucleotide sequence. For instance, a promoter is operably associated with a nucleotide sequence if the promoter effects the transcription or expression of said nucleotide sequence. Those skilled in the art will appreciate that the control sequences (*e*.*g*., promoter) need not be contiguous with the nucleotide sequence to which it is operably associated, as long as the control sequences function to direct the expression thereof. Thus, for example, intervening untranslated, yet transcribed, nucleic acid sequences can be present between a promoter and the nucleotide sequence, and the promoter can still be considered "operably linked" to the nucleotide sequence.

As used herein, the term "linked," or "fused" in reference to polypeptides, refers to the attachment of one polypeptide to another. A polypeptide may be linked or fused to another polypeptide (at the N-terminus or the C-terminus) directly (e.g., via a peptide bond) or through a linker (e.g., a peptide linker).

The term "linker" in reference to polypeptides is art-recognized and refers to a chemical group, or a molecule linking two molecules or moieties, e.g., two polypeptides (e.g., domains) of a fusion protein, such as, for example, a Type V CRISPR-Cas effector protein and a peptide tag and/or a polypeptide of interest. A linker may be comprised of a single linking molecule (e.g., a single amino acid) or may comprise more than one linking molecule. In some embodiments, the linker can be an organic molecule, group, polymer, or chemical moiety such as a bivalent organic moiety. In some embodiments, the linker may be an amino acid or it may be a peptide. In some embodiments, the linker is a peptide.

In some embodiments, a peptide linker useful with this invention may be about 2 to about 100 or more amino acids in length, for example, about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more amino acids in length (e.g., about 2 to about 40, about 2 to about 50, about 2 to about 60, about 4 to about 40, about 4 to about 50, about 4 to about 60, about 5 to about 40, about 5 to about 50, about 5 to about 60, about 9 to about 40, about 9 to about 50, about 9 to about 60, about 10 to about 40, about 10 to about 50, about 10 to about 60, or about 2, 3, 4, 5, 6, 7, 8, 9, 10, 11, 12, 13, 14, 15, 16, 17, 18, 19, 20, 21, 22, 23, 24, 25 amino acids to about 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more amino acids in length (e.g., about 105, 110, 115, 120, 130, 140 150 or more amino acids in length). In some embodiments, a peptide linker may be a GS linker.

In some embodiments, two or more polynucleotide molecules may be linked by a linker that can be an organic molecule, group, polymer, or chemical moiety such as a bivalent organic moiety. A polynucleotide may be linked or fused to another polynucleotide (at the 5' end or the 3' end) via a covalent or non-covenant linkage or binding, including e.g., Watson-Crick base-pairing, or through one or more linking nucleotides. In some embodiments, a polynucleotide motif of a certain structure may be inserted within another polynucleotide sequence (e.g. extension of the hairpin structure in guide RNA). In some embodiments, the linking nucleotides may be naturally occurring nucleotides. In some embodiments, the linking nucleotides may be non-naturally occurring nucleotides.

A "promoter" is a nucleotide sequence that controls or regulates the transcription of a nucleotide sequence (*e.g.*, a coding sequence) that is operably associated with the promoter. The coding sequence controlled or regulated by a promoter may encode a polypeptide and/or a functional RNA. Typically, a "promoter" refers to a nucleotide sequence that contains a binding site for RNA polymerase II and directs the initiation of transcription. In general, promoters are found 5', or upstream, relative to the start of the coding region of the corresponding coding sequence. A promoter may comprise other elements that act as regulators of gene expression; e.g., a promoter region. These include a TATA box consensus sequence, and often a CAAT box consensus sequence (Breathnach and Chambon, (1981) Annu. Rev. Biochem. 50:349). In plants, the CAAT box may be substituted by the AGGA box (Messing et al., (1983) in Genetic Engineering of Plants, T. Kosuge, C. Meredith and A. Hollaender (eds.), Plenum Press, pp. 211-227). In some embodiments, a promoter region may comprise at least one intron (e.g., **SEQ ID NO:1** or **SEQ ID NO:2).**

Promoters useful with this invention can include, for example, constitutive, inducible, temporally regulated, developmentally regulated, chemically regulated, tissue-preferred and/or tissue-specific promoters for use in the preparation of recombinant nucleic acid molecules, e.g., "synthetic nucleic acid constructs" or "protein-RNA complex." These various types of promoters are known in the art.

The choice of promoter may vary depending on the temporal and spatial requirements for expression, and also may vary based on the host cell to be transformed. Promoters for many different organisms are well known in the art. Based on the extensive knowledge present in the art, the appropriate promoter can be selected for the particular host organism of interest. Thus, for example, much is known about promoters upstream of highly constitutively expressed genes in model organisms and such knowledge can be readily accessed and implemented in other systems as appropriate.

In some embodiments, a promoter functional in a plant may be used with the constructs of this invention. Non-limiting examples of a promoter useful for driving expression in a plant include the promoter of the RubisCo small subunit gene 1 (PrbcS 1), the promoter of the actin gene (Pactin), the promoter of the nitrate reductase gene (Pnr) and the promoter of duplicated carbonic anhydrase gene 1 (Pdca1) (*See*, Walker et al. Plant Cell Rep. 23:727-735 (2005); Li et al. Gene 403:132-142 (2007); Li et al. MolBiol. Rep. 37:1143-1154 (2010)). PrbcS1 and Pactin are constitutive promoters and Pnr and Pdca1 are inducible promoters. Pnr is induced by nitrate and repressed by ammonium (Li et al. Gene 403:132-142 (2007)) and Pdca1 is induced by salt (Li et al. Mol Biol. Rep. 37:1143-1154 (2010)).

Examples of constitutive promoters useful for plants include, but are not limited to, cestrum virus promoter (cmp) (U.S. Patent No. 7,166,770), the rice actin 1 promoter (Wang et al. (1992)Mol. Cell. Biol. 12:3399-3406; as well as US Patent No. 5,641,876), CaMV 35S promoter (Odell et al. (1985) Nature 313:810-812), CaMV 19S promoter (Lawton et al. (1987) Plant Mol. Biol. 9:315-324), nos promoter (Ebert et al. (1987) Proc. Natl. Acad. Sci USA 84:5745-5749), Adh promoter (Walker et al. (1987) Proc. Natl. Acad. Sci. USA 84:6624-6629), sucrose synthase promoter (Yang & Russell (1990) Proc. Natl. Acad. Sci. USA 87:4144-4148), and the ubiquitin promoter. The constitutive promoter derived from ubiquitin accumulates in many cell types. Ubiquitin promoters have been cloned from several plant species for use in transgenic plants, for example, sunflower (Binet et al., 1991. Plant Science 79: 87-94), maize (Christensen et al., 1989. Plant Molec. Biol. 12: 619-632), and arabidopsis (Norris et al. 1993. Plant Molec. Biol. 21:895-906). The maize ubiquitin promoter (*UbiP*) has been developed in transgenic monocot systems and its sequence and vectors constructed for monocot transformation are disclosed in the European patent publication EP0342926. The ubiquitin promoter is suitable for the expression of the nucleotide sequences of the invention in transgenic plants, especially monocotyledons. Further, the promoter expression cassettes described by McElroy et al. (Mol. Gen. Genet. 231: 150-160 (1991)) can be easily modified for the expression of the nucleotide sequences of the invention and are particularly suitable for use in monocotyledonous hosts.

In some embodiments, tissue specific/tissue preferred promoters can be used for expression of a heterologous polynucleotide in a plant cell. Tissue specific or preferred expression patterns include, but are not limited to, green tissue specific or preferred, root specific or preferred, stem specific or preferred, flower specific or preferred or pollen specific or preferred. Promoters suitable for expression in green tissue include many that regulate genes involved in photosynthesis and many of these have been cloned from both monocotyledons and dicotyledons. In one embodiment, a promoter useful with the invention is the maize PEPC promoter from the phosphoenol carboxylase gene (Hudspeth & Grula, Plant Molec. Biol. 12:579-589 (1989)). Non-limiting examples of tissue-specific promoters include those associated with genes encoding the seed storage proteins (such as β-conglycinin, cruciferin, napin and phaseolin), zein or oil body proteins (such as oleosin), or proteins involved in fatty acid biosynthesis (including acyl carrier protein, stearoyl-ACP desaturase and fatty acid desaturases (fad 2-1)), and other nucleic acids expressed during embryo development (such as Bce4, *see*, *e.g.*, Kridl et al. (1991) Seed Sci. Res. 1:209-219; as well as EP Patent No. 255378). Tissue-specific or tissue-preferential promoters useful for the expression of the nucleotide sequences of the invention in plants, particularly maize, include but are not limited to those that direct expression in root, pith, leaf or pollen. Such promoters are disclosed, for example, in WO 93/07278, incorporated by reference herein for its disclosure of promoters. Other non-limiting examples of tissue specific or tissue preferred promoters useful with the invention the cotton rubisco promoter disclosed in US Patent 6,040,504; the rice sucrose synthase promoter disclosed in US Patent 5,604,121; the root specific promoter described by de Framond (FEBS 290:103-106 (1991); European patent EP 0452269 to Ciba- Geigy); the stem specific promoter described in U.S. Patent 5,625,136 (to Ciba-Geigy) and which drives expression of the maize trpA gene; the cestrum yellow leaf curling virus promoter disclosed in WO 01/73087; and pollen specific or preferred promoters including, but not limited to, ProOsLPS10 and ProOsLPS11 from rice (Nguyen et al. Plant Biotechnol. Reports 9(5):297-306 (2015)), ZmSTK2_USP from maize (Wang et al. Genome 60(6):485-495 (2017)), LAT52 and LAT59 from tomato (Twell et al. Development 109(3):705-713 (1990)), Zm13 (U.S. Patent No. 10,421,972), PLA₂-δ promoter from arabidopsis (U.S. Patent No. 7,141,424), and/or the ZmC5 promoter from maize (International PCT Publication No. WO1999/042587.

Additional examples of plant tissue-specific/tissue preferred promoters include, but are not limited to, the root hair-specific ***cis***-elements (RHEs) (Kim et al. The Plant Cell 18:2958-2970 (2006)), the root-specific promoters *RCc3* (Jeong et al. Plant Physiol. 153:185-197 (2010)) and RB7 (U.S. Patent No. 5459252), the lectin promoter (Lindstrom et al. (1990) Der. Genet. 11:160-167; and Vodkin (1983) Prog. Clin. Biol. Res. 138:87-98), corn alcohol dehydrogenase 1 promoter (Dennis et al. (1984) Nucleic Acids Res. 12:3983-4000), S-adenosyl-L-methionine synthetase (SAMS) (Vander Mijnsbrugge et al. (1996) Plant and Cell Physiology, 37(8): 1108-1115), corn light harvesting complex promoter (Bansal et al. (1992) Proc. Natl. Acad. Sci. USA 89:3654-3658), corn heat shock protein promoter (O'Dell et al. (1985) EMBO J. 5:451-458; and Rochester et al. (1986) EMBO J. 5:451-458), pea small subunit RuBP carboxylase promoter (Cashmore, "Nuclear genes encoding the small subunit of ribulose-l,5-bisphosphate carboxylase" pp. 29-39 In: Genetic Engineering of Plants (Hollaender ed., Plenum Press 1983; and Poulsen et al. (1986) Mol. Gen. Genet. 205:193-200), Ti plasmid mannopine synthase promoter (Langridge et al. (1989) Proc. Natl. Acad. Sci. USA 86:3219-3223), Ti plasmid nopaline synthase promoter (Langridge *et al.* (1989), *supra*), petunia chalcone isomerase promoter (van Tunen et al. (1988) EMBO J. 7:1257-1263), bean glycine rich protein 1 promoter (Keller et al. (1989) Genes Dev. 3:1639-1646), truncated CaMV 35S promoter (O'Dell et al. (1985) Nature 313:810-812), potato patatin promoter (Wenzler et al. (1989) Plant Mol. Biol. 13:347-354), root cell promoter (Yamamoto et al. (1990) Nucleic Acids Res. 18:7449), maize zein promoter (Kriz et al. (1987) Mol. Gen. Genet. 207:90-98; Langridge et al. (1983) Cell 34:1015-1022; Reina et al. (1990) Nucleic Acids Res. 18:6425; Reina et al. (1990) Nucleic Acids Res. 18:7449; and Wandelt et al. (1989) Nucleic Acids Res. 17:2354), globulin-1 promoter (Belanger et al. (1991) Genetics 129:863-872), α-tubulin cab promoter (Sullivan et al. (1989) Mol. Gen. Genet. 215:431-440), PEPCase promoter (Hudspeth & Grula (1989) Plant Mol. Biol. 12:579-589), R gene complex-associated promoters (Chandler et al. (1989) Plant Cell 1:1175-1183), and chalcone synthase promoters (Franken et al. (1991) EMBO J. 10:2605-2612).

Useful for seed-specific expression is the pea vicilin promoter (Czako et al. (1992) Mol. Gen. Genet. 235:33-40; as well as the seed-specific promoters disclosed in U.S. Patent No. 5,625,136. Useful promoters for expression in mature leaves are those that are switched at the onset of senescence, such as the SAG promoter from *Arabidopsis* (Gan et al. (1995) Science 270:1986-1988).

In addition, promoters functional in chloroplasts can be used. Non-limiting examples of such promoters include the bacteriophage T3 gene 9 5' UTR and other promoters disclosed in U.S. Patent No. 7,579,516. Other promoters useful with the invention include but are not limited to the S-E9 small subunit RuBP carboxylase promoter and the Kunitz trypsin inhibitor gene promoter (Kti3).

Additional regulatory elements useful with this invention include, but are not limited to, introns, enhancers, termination sequences and/or 5' and 3' untranslated regions.

An intron useful with this invention can be an intron identified in and isolated from a plant and then inserted into an expression cassette to be used in transformation of a plant. As would be understood by those of skill in the art, introns can comprise the sequences required for self-excision and are incorporated into nucleic acid constructs/expression cassettes in frame. An intron can be used either as a spacer to separate multiple protein-coding sequences in one nucleic acid construct, or an intron can be used inside one protein-coding sequence to, for example, stabilize the mRNA. If they are used within a protein-coding sequence, they are inserted "in-frame" with the excision sites included. Introns may also be associated with promoters to improve or modify expression. As an example, a promoter/intron combination useful with this invention includes but is not limited to that of the maize Ubi1 promoter and intron.

Non-limiting examples of introns useful with the present invention include introns from the ADHI gene (e.g., *Adh1-S* introns 1, 2 and 6), the ubiquitin gene (Ubi1), the RuBisCO small subunit (rbcS) gene, the RuBisCO large subunit (rbcL) gene, the actin gene (e.g., *actin-1* intron), the pyruvate dehydrogenase kinase gene (pdk), the nitrate reductase gene (nr), the duplicated carbonic anhydrase gene 1 (Tdca1), the psbA gene, the atpA gene, or any combination thereof.

In some embodiments, a polynucleotide and/or a nucleic acid construct of the invention can be an "expression cassette" or can be comprised within an expression cassette. As used herein, "expression cassette" means a recombinant nucleic acid molecule comprising, for example, a nucleic acid construct of the invention (e.g., a polynucleotide encoding a Type V CRISPR-Cas effector protein , a polynucleotide encoding a Type V CRISPR-Cas fusion protein, a polynucleotide encoding a deaminase (e.g., a cytosine deaminase and/or an adenine deaminase), a polynucleotide encoding a deaminase fusion protein, a polynucleotide encoding a peptide tag, a polynucleotide encoding an affinity polypeptide, a recruiting guide nucleic acid and/or a guide nucleic acid), wherein the nucleic acid construct is operably associated with at least a control sequence (e.g., a promoter). Thus, some embodiments of the invention provide expression cassettes designed to express, for example, a nucleic acid construct of the invention. When an expression cassette comprises more than one polynucleotide, the polynucleotides may be operably linked to a single promoter that drives expression of all of the polynucleotides or the polynucleotides may be operably linked to one or more different promoters (e.g., three polynucleotides may be driven by one, two or three promoters in any combination). Thus, for example, a polynucleotide encoding a Type V CRISPR-Cas fusion protein, a polynucleotide encoding a deaminase fusion protein, and a guide nucleic acid comprised in an expression cassette may each be operably associated with a single promoter or they may be operably associated with separate promoters (e.g., two or three promoters) in any combination. As another example, a polynucleotide encoding a Type V CRISPR-Cas effector protein, a polynucleotide encoding a deaminase fusion protein, and a recruiting guide nucleic acid comprised in an expression cassette may each be operably associated with a single promoter or they may be operably associated with separate promoters (e.g., two or three promoters) in any combination.

In some embodiments, an expression cassette comprising the polynucleotides/nucleic acid constructs of the invention may be optimized for expression in an organism (e.g., an animal, a plant, a bacterium and the like).

An expression cassette comprising a nucleic acid construct of the invention may be chimeric, meaning that at least one of its components is heterologous with respect to at least one of its other components (e.g., a promoter from the host organism operably linked to a polynucleotide of interest to be expressed in the host organism, wherein the polynucleotide of interest is from a different organism than the host or is not normally found in association with that promoter). An expression cassette may also be one that is naturally occurring but has been obtained in a recombinant form useful for heterologous expression.

An expression cassette can optionally include a transcriptional and/or translational termination region (i.e., termination region) and/or an enhancer region that is functional in the selected host cell. A variety of transcriptional terminators and enhancers are known in the art and are available for use in expression cassettes. Transcriptional terminators are responsible for the termination of transcription and correct mRNA polyadenylation. A termination region and/or the enhancer region may be native to the transcriptional initiation region, may be native to a gene encoding a CRISPR-Cas effector protein or a gene encoding a deaminase, may be native to a host cell, or may be native to another source (*e*.*g*., foreign or heterologous to the promoter, to a gene encoding the CRISPR-Cas effector protein or a gene encoding the deaminase, to a host cell, or any combination thereof).

An expression cassette of the invention also can include a polynucleotide encoding a selectable marker, which can be used to select a transformed host cell. As used herein, "selectable marker" means a polynucleotide sequence that when expressed imparts a distinct phenotype to the host cell expressing the marker and thus allows such transformed cells to be distinguished from those that do not have the marker. Such a polynucleotide sequence may encode either a selectable or screenable marker, depending on whether the marker confers a trait that can be selected for by chemical means, such as by using a selective agent (*e*.*g*., an antibiotic and the like), or on whether the marker is simply a trait that one can identify through observation or testing, such as by screening (*e*.*g*., fluorescence). Many examples of suitable selectable markers are known in the art and can be used in the expression cassettes described herein.

The expression cassettes, the nucleic acid molecules/constructs and polynucleotide sequences described herein can be used in connection with vectors. The term "vector" refers to a composition for transferring, delivering or introducing a nucleic acid (or nucleic acids) into a cell. A vector comprises a nucleic acid construct comprising the nucleotide sequence(s) to be transferred, delivered or introduced. Vectors for use in transformation of host organisms are well known in the art. Non-limiting examples of general classes of vectors include viral vectors, plasmid vectors, phage vectors, phagemid vectors, cosmid vectors, fosmid vectors, bacteriophages, artificial chromosomes, minicircles, or Agrobacterium binary vectors in double or single stranded linear or circular form which may or may not be self transmissible or mobilizable. In some embodiments, a viral vector can include, but is not limited, to a retroviral, lentiviral, adenoviral, adeno-associated, or herpes simplex viral vector. A vector as defined herein can transform a prokaryotic or eukaryotic host either by integration into the cellular genome or exist extrachromosomally (e.g. autonomous replicating plasmid with an origin of replication). Additionally included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal cells). In some embodiments, the nucleic acid in the vector is under the control of, and operably linked to, an appropriate promoter or other regulatory elements for transcription in a host cell. The vector may be a bi-functional expression vector which functions in multiple hosts. In the case of genomic DNA, this may contain its own promoter and/or other regulatory elements and in the case of cDNA this may be under the control of an appropriate promoter and/or other regulatory elements for expression in the host cell. Accordingly, a nucleic acid construct of this invention and/or expression cassettes comprising the same may be comprised in vectors as described herein and as known in the art.

As used herein, "contact," "contacting," "contacted," and grammatical variations thereof, refer to placing the components of a desired reaction together under conditions suitable for carrying out the desired reaction (e.g., transformation, transcriptional control, genome editing, nicking, and/or cleavage). Thus, for example, a target nucleic acid may be contacted with a nucleic acid construct of the invention encoding, for example, Type V CRISPR-Cas fusion protein, a deaminase fusion protein and a guide nucleic acid, under conditions whereby the Type V CRISPR-Cas fusion protein is expressed, and the Type V CRISPR-Cas fusion protein forms a complex with the guide nucleic acid, the complex hybridizes to the target nucleic acid, and the deaminase fusion protein is recruited to the Type V CRISPR-Cas effector protein (and thus, to the target nucleic acid), thereby modifying the target nucleic acid. In some embodiments, a Type V CRISPR-Cas protein (optionally a Type V CRISPR-Cas fusion protein), a guide nucleic acid, and a deaminase (optionally a deaminase fusion protein) contact a target nucleic acid to thereby modify the nucleic acid. In some embodiments, the Type V CRISPR-Cas protein, guide nucleic acid, and/or deaminase may be in the form of a complex (e.g., a ribonucleoprotein such as an assembled ribonucleoprotein complex) and the complex contacts the target nucleic acid. In some embodiments, the complex or a component thereof (e.g., the guide nucleic acid) hybridizes to the target nucleic acid and thereby the target nucleic acid is modified (e.g., via action of the Type V CRISPR-Cas protein and/or deaminase). In some embodiments, a deaminase or deaminase fusion protein and the Type V CRISPR-Cas effector protein localize at the target nucleic acid, optionally through covalent and/or non-covalent interactions.

As used herein, "modifying" or "modification" in reference to a target nucleic acid includes editing (e.g., mutating), covalent modification, exchanging/substituting nucleic acids/nucleotide bases, deleting, cleaving, nicking, and/or transcriptional control of a target nucleic acid.

"Recruit," "recruiting" or "recruitment" as used herein refer to attracting one or more polypeptide(s) or polynucleotide(s) to another polypeptide or polynucleotide (e.g., to a particular location in a genome) using protein-protein interactions, RNA-protein interactions, and/or chemical interactions. Protein-protein interactions can include, but are not limited to, peptide tags (e.g., epitopes, multimerized epitopes) and corresponding affinity polypeptides, RNA recruiting motifs and corresponding affinity polypeptides, and/or chemical interactions. Example chemical interactions that may be useful with polypeptides and polynucleotides for the purpose of recruitment can include, but are not limited to, rapamycin-inducible dimerization of FRB - FKBP; Biotin-streptavidin interaction; SNAP tag (Hussain et al. Curr Pharm Des. 19(30):5437-42 (2013)); Halo tag (Los et al. ACS Chem Biol. 3(6):373-82 (2008)); CLIP tag (Gautier et al. Chemistry & Biology 15:128-136 (2008)); DmrA-DmrC heterodimer induced by a compound (Tak et al. Nat Methods 14(12): 1163-1166 (2017)); Bifunctional ligand approaches (fuse two protein-binding chemicals together) (Voß et al. Curr Opin Chemical Biology 28:194-201 (2015)) (e.g. dihyrofolate reductase (DHFR) (Kopyteck et al. Cell Cehm Biol 7(5):313-321 (2000)).

"Introducing," "introduce," "introduced" (and grammatical variations thereof) in the context of a polynucleotide of interest means presenting a nucleotide sequence of interest (e.g., polynucleotide, a nucleic acid construct, and/or a guide nucleic acid) to a host organism or cell of said organism (e.g., host cell; e.g., a plant cell) in such a manner that the nucleotide sequence gains access to the interior of a cell. Thus, for example, a nucleic acid construct of the invention encoding a Type V CRISPR-Cas fusion protein and a deaminase fusion protein as described herein and guide nucleic acid may be introduced into a cell of an organism, thereby transforming the cell with the Type V CRISPR-Cas effector protein fusion protein, the deaminase fusion protein and the guide nucleic acid.

The term "transformation" as used herein refers to the introduction of a heterologous nucleic acid into a cell. Transformation of a cell may be stable or transient. Thus, in some embodiments, a host cell or host organism may be stably transformed with a polynucleotide/nucleic acid molecule of the invention. In some embodiments, a host cell or host organism may be transiently transformed with a nucleic acid construct of the invention.

"Transient transformation" in the context of a polynucleotide means that a polynucleotide is introduced into the cell and does not integrate into the genome of the cell.

By "stably introducing" or "stably introduced" in the context of a polynucleotide introduced into a cell is intended that the introduced polynucleotide is stably incorporated into the genome of the cell, and thus the cell is stably transformed with the polynucleotide.

"Stable transformation" or "stably transformed" as used herein means that a nucleic acid molecule is introduced into a cell and integrates into the genome of the cell. As such, the integrated nucleic acid molecule is capable of being inherited by the progeny thereof, more particularly, by the progeny of multiple successive generations. "Genome" as used herein includes the nuclear and the plastid genome, and therefore includes integration of the nucleic acid into, for example, the chloroplast or mitochondrial genome. Stable transformation as used herein can also refer to a transgene that is maintained extrachromasomally, for example, as a minichromosome or a plasmid.

Transient transformation may be detected by, for example, an enzyme-linked immunosorbent assay (ELISA) or Western blot, which can detect the presence of a peptide or polypeptide encoded by one or more transgene introduced into an organism. Stable transformation of a cell can be detected by, for example, a Southern blot hybridization assay of genomic DNA of the cell with nucleic acid sequences which specifically hybridize with a nucleotide sequence of a transgene introduced into an organism (e.g., a plant). Stable transformation of a cell can be detected by, for example, a Northern blot hybridization assay of RNA of the cell with nucleic acid sequences which specifically hybridize with a nucleotide sequence of a transgene introduced into a host organism. Stable transformation of a cell can also be detected by, *e.g.*, a polymerase chain reaction (PCR) or other amplification reactions as are well known in the art, employing specific primer sequences that hybridize with target sequence(s) of a transgene, resulting in amplification of the transgene sequence, which can be detected according to standard methods Transformation can also be detected by direct sequencing and/or hybridization protocols well known in the art.

Accordingly, in some embodiments, nucleotide sequences, polynucleotides, nucleic acid constructs, and/or expression cassettes of the invention may be expressed transiently and/or they can be stably incorporated into the genome of the host organism. Thus, in some embodiments, a nucleic acid construct of the invention may be transiently introduced into a cell with a guide nucleic acid and as such, no DNA maintained in the cell.

A nucleic acid construct of the invention can be introduced into a cell by any method known to those of skill in the art. In some embodiments of the invention, transformation of a cell comprises nuclear transformation. In other embodiments, transformation of a cell comprises plastid transformation (e.g., chloroplast transformation). In still further embodiments, the recombinant nucleic acid construct of the invention can be introduced into a cell via conventional breeding techniques.

Procedures for transforming both eukaryotic and prokaryotic organisms are well known and routine in the art and are described throughout the literature (*See*, for example, Jiang et al. 2013. Nat. Biotechnol. 31:233-239; Ran et al. Nature Protocols 8:2281-2308 (2013)).

A nucleotide sequence therefore can be introduced into a host organism or its cell in any number of ways that are well known in the art. The methods of the invention do not depend on a particular method for introducing one or more nucleotide sequences into the organism, only that they gain access to the interior of at least one cell of the organism. Where more than one nucleotide sequence is to be introduced, they can be assembled as part of a single nucleic acid construct, or as separate nucleic acid constructs, and can be located on the same or different nucleic acid constructs. Accordingly, the nucleotide sequences can be introduced into the cell of interest in a single transformation event, and/or in separate transformation events, or, alternatively, where relevant, a nucleotide sequence can be incorporated into a plant, for example, as part of a breeding protocol.

The present invention is directed to improved base editing nucleic acid constructs. In some embodiments, the present invention provides a nucleic acid construct comprising: (a) a Type V CRISPR-Cas fusion protein comprising a Type V CRISPR-Cas effector protein fused to a peptide tag; (b) a deaminase fusion protein comprising a deaminase fused to an affinity polypeptide that is capable of binding to the peptide tag; and (c) a guide nucleic acid. In some embodiments, the present invention provides a nucleic acid construct comprising: (a) a Cas12a fusion protein comprising a Cas12a effector protein fused to a peptide tag; (b) a deaminase fusion protein comprising a deaminase fused to an affinity polypeptide that binds to the peptide tag; and (c) a guide nucleic acid.

In some embodiments, the Cas12a (Cpfl) effector protein may be a LbCpfl [*Lachnospiraceae* bacterium], AsCpf1 [*Acidaminococcus* sp.], BpCpf1 [*Butyrivibrio proteoclasticus*], CMtCpf1 [*Candidates Methanoplasma termitum* ], EeCpf1 [*Eubacterium eligens*], FnCpf1 (*Francisella novicida* U112), Lb2Cpf1 [*Lachnospiraceae* bacterium], >Lb3Cpf1 [*Lachnospiraceae* bacterium], LiCpf1 [*Leptospira inadai*], MbCpf1 [*Moraxella bovoculi* 237], PbCpf1 [Parcubacteria bacterium GWC2011_GWC2_44_17], PcCpf1 [*Porphyromonas crevioricanis*], PdCpf1 [*Prevotella disiens*], PeCpf1 [*Peregrinibacteria* bacterium GW2011_GWA_33_10], PmCpf1 [*Porphyromonas macacae*], and/or a SsCpf1 [*Smithella* sp. SC_K08D17] (e.g., having a sequence of any one of **SEQ ID NOs:3-22).** In some embodiments, the Cas12a effector protein may be a *Lachnospiraceae* bacterium ND2006 Cas12a (LbCas12a)(LbCpf1) (e.g., having a sequence of any one of **SEQ ID NOs:3** and **9-11),** an *Acidaminococcus sp.* Cpfl (AsCas12a) (AsCpf1) (e.g., having a sequence of any one **of SEQ ID NO:4)** and/or enAsCas12a (e.g., having a sequence of any one of **SEQ ID NOs:20-22).**

In some embodiments, the present invention provides a nucleic acid construct comprising: (a) a Type V CRISPR-Cas fusion protein comprising a Type V CRISPR-Cas effector protein fused to an affinity polypeptide that is capable of binding to a peptide tag; (b) a deaminase fusion protein comprising a deaminase fused to the peptide tag; and (c) a guide nucleic acid. In some embodiments, the present invention provides a nucleic acid construct comprising: (a) a Cas12a fusion protein comprising a Cas12a effector protein fused to an affinity polypeptide that binds to a peptide tag; (b) a deaminase fusion protein comprising a deaminase fused to the peptide tag; and (c) a guide nucleic acid.

In some embodiments, a nucleic acid construct is provided comprising:(a) a Type V CRISPR-Cas effector protein ; (b) a recruiting guide nucleic acid comprising a guide nucleic acid linked to an RNA recruiting motif; and (c) a deaminase fusion protein comprising a deaminase fused to an affinity polypeptide that binds to the RNA recruiting motif. In some embodiments, a nucleic acid construct is provided comprising:(a) a Cas12a effector protein ; (b) a recruiting guide nucleic acid comprising a guide nucleic acid linked to an RNA recruiting motif; and (c) a deaminase fusion protein comprising a deaminase fused to an affinity polypeptide that binds to the RNA recruiting motif.

In some embodiments, a Type V Clustered Regularly Interspaced Short Palindromic Repeats (CRISPR)-associated (Cas) (CRISPR-Cas) system is provided comprising: (a) a Type V CRISPR-Cas fusion protein comprising a Type V CRISPR-Cas effector protein fused to a peptide tag; (b) a deaminase fusion protein comprising a deaminase fused to an affinity polypeptide that binds to the peptide tag; and (c) a guide nucleic acid comprising a spacer sequence and a repeat sequence, wherein the guide nucleic acid is capable of forming a complex with the Type V CRISPR-Cas effector protein of the Type V CRISPR-Cas fusion protein and the spacer sequence is capable of hybridizing to a target nucleic acid, thereby guiding the Type V CRISPR-Cas fusion protein to the target nucleic acid, and wherein the deaminase fusion protein is recruited to the Type V CRISPR-Cas fusion protein and target nucleic acid by the binding of the affinity polypeptide to the peptide tag that is fused to the Type V CRISPR-Cas fusion protein, whereby the system is capable of modifying (e.g., cleaving or editing) the target nucleic acid. In some embodiments, a Type V Clustered Regularly Interspaced Short Palindromic Repeats (CRISPR)-associated (Cas) (CRISPR-Cas) system is provided comprising: (a) a Cas12a fusion protein comprising a Cas12a effector protein fused to a peptide tag; (b) a deaminase fusion protein comprising a deaminase fused to an affinity polypeptide that binds to the peptide tag; and (c) a guide nucleic acid comprising a spacer sequence and a repeat sequence, wherein the guide nucleic acid is capable of forming a complex with the Cas 12a effector protein of the Cas 12a fusion protein and the spacer sequence is capable of hybridizing to a target nucleic acid, thereby guiding the Cas12a fusion protein to the target nucleic acid, and wherein the deaminase fusion protein is recruited to the Cas12a fusion protein and target nucleic acid by the binding of the affinity polypeptide to the peptide tag that is fused to the Cas12a fusion protein, whereby the system is capable of modifying (e.g., cleaving or editing) the target nucleic acid.

In some embodiments, the present invention provides a Type V Clustered Regularly Interspaced Short Palindromic Repeats (CRISPR)-associated (Cas) (CRISPR-Cas) system comprising: (a) a Type V CRISPR-Cas effector protein ; (b) a recruiting guide nucleic acid comprising a guide nucleic acid linked to an RNA recruiting motif, and (c) a deaminase fusion protein comprising a deaminase fused to an affinity polypeptide that binds to the RNA recruiting motif, wherein the recruiting guide nucleic acid comprises a spacer sequence and a repeat sequence, wherein the guide nucleic acid is capable of forming a complex with the Type V CRISPR-Cas effector protein and the spacer sequence is capable of hybridizing to a target nucleic acid, thereby guiding the Type V CRISPR-Cas effector protein to the target nucleic acid, and wherein the deaminase fusion protein is recruited to the Type V CRISPR-Cas effector protein and target nucleic acid by the binding of the affinity polypeptide to the RNA recruiting motif that is fused to the recruiting guide nucleic acid, whereby the system is capable of modifying (e.g., cleaving or editing) the target nucleic acid. In some embodiments, the present invention provides a Type V Clustered Regularly Interspaced Short Palindromic Repeats (CRISPR)-associated (Cas) (CRISPR-Cas) system comprising: (a) a Cas12a effector protein; (b) a recruiting guide nucleic acid comprising a guide nucleic acid linked to an RNA recruiting motif, and (c) a deaminase fusion protein comprising a deaminase fused to an affinity polypeptide that binds to the RNA recruiting motif, wherein the recruiting guide nucleic acid comprises a spacer sequence and a repeat sequence, wherein the guide nucleic acid is capable of forming a complex with the Cas12a effector protein and the spacer sequence is capable of hybridizing to a target nucleic acid, thereby guiding the Cas12a effector protein to the target nucleic acid, and wherein the deaminase fusion protein is recruited to the Cas 12a effector protein and target nucleic acid by the binding of the affinity polypeptide to the RNA recruiting motif that is fused to the recruiting guide nucleic acid, whereby the system is capable of modifying (e.g., cleaving or editing) the target nucleic acid.

In some embodiments, a nucleic acid construct of the invention (e.g., a polynucleotide encoding a Type V CRISPR-Cas effector protein, a polynucleotide encoding a Type V CRISPR-Cas fusion protein, a polynucleotide encoding a deaminase, a polynucleotide encoding a deaminase fusion protein, a polynucleotide encoding a peptide tag, a polynucleotide encoding an affinity polypeptide, an RNA recruiting motif, a recruiting guide nucleic acid and/or a guide nucleic acid and/or expression cassettes and/or vectors comprising the same) may be operably linked to at least one regulatory sequence, optionally, wherein the at least one regulatory sequence may be codon optimized for expression in a plant. In some embodiments, the at least one regulatory sequence may be, for example, a promoter, an operon, a terminator, or an enhancer. In some embodiments, the at least one regulatory sequence may be a promoter. In some embodiments, the regulatory sequence may be an intron. In some embodiments, the at least one regulatory sequence may be, for example, a promoter operably associated with an intron or a promoter region comprising an intron. In some embodiments, the at least one regulatory sequence may be, for example a ubiquitin promoter and its associated intron (e.g., *Medicago truncatula* and/or *Zea mays* and their associated introns). In some embodiments, the at least one regulatory sequence may be a terminator nucleotide sequence and/or an enhancer nucleotide sequence.

In some embodiments, a nucleic acid construct of the invention may be operably associated with a promoter region, wherein the promoter region comprises an intron, optionally wherein the promoter region may be a ubiquitin promoter and intron (e.g., a *Medicago* or a maize ubiquitin promoter and intron, e.g., **SEQ ID NO:1** or **SEQ ID NO:2).** In some embodiments, the nucleic acid construct of the invention that is operably associated with a promoter region comprising an intron may be codon optimized for expression in a plant.

In some embodiments, a nucleic acid construct of the invention may further encode one or more polypeptides of interest, optionally wherein the one or more polypeptides of interest may be codon optimized for expression in a plant.

A polypeptide of interest useful with this invention can include, but is not limited to, a polypeptide or protein domain having deaminase activity, nickase activity, recombinase activity, transposase activity, methylase activity, glycosylase (DNA glycosylase) activity, glycosylase inhibitor activity (e.g., uracil-DNA glycosylase inhibitor (UGI)), demethylase activity, transcription activation activity, transcription repression activity, transcription release factor activity, histone modification activity, nuclease activity, single-strand RNA cleavage activity, double-strand RNA cleavage activity, restriction endonuclease activity (e.g., Fok1), nucleic acid binding activity, methyltransferase activity, DNA repair activity, DNA damage activity, dismutase activity, alkylation activity, depurination activity, oxidation activity, pyrimidine dimer forming activity, integrase activity, transposase activity, polymerase activity, ligase activity, helicase activity, a nuclear localization sequence or activity, and/or photolyase activity. In some embodiments, the polypeptide of interest is a Fok1 nuclease, or a uracil-DNA glycosylase inhibitor. When encoded in a nucleic acid (polynucleotide, expression cassette, and/or vector) the encoded polypeptide or protein domain may be codon optimized for expression in an organism. In some embodiments, a polypeptide of interest may be linked to a Type V CRISPR-Cas effector protein to provide a Type V CRISPR-Cas fusion protein comprising a Type V CRISPR-Cas effector protein and a polypeptide of interest. In some embodiments, a Type V CRISPR-Cas fusion protein that comprises a Type V CRISPR-Cas effector protein linked to a peptide tag or an affinity polypeptide may also be linked to a polypeptide of interest (e.g., a Type V CRISPR-Cas effector protein may be, for example, linked to both a peptide tag (or an affinity polypeptide) and, for example, a polypeptide of interest, e.g., a UGI). In some embodiments, a polypeptide of interest may be a uracil glycosylase inhibitor (e.g., uracil-DNA glycosylase inhibitor (UGI)).

In some embodiments, a nucleic acid construct of the invention encoding a Type V CRISPR-Cas fusion protein, a deaminase fusion protein and comprising a guide nucleic acid may further encode a polypeptide of interest, optionally wherein the polypeptide of interest may be codon optimized for expression in an organism. In some embodiments, a nucleic acid construct of the invention encoding a Type V CRISPR-Cas effector protein, a deaminase fusion protein and comprising a recruiting guide nucleic acid may further encode a polypeptide of interest, optionally wherein the polypeptide of interest may be codon optimized for expression in an organism (e.g., a plant).

As used herein, a "Type V CRISPR-Cas effector protein" or "Type V Clustered Regularly Interspaced Short Palindromic Repeats (CRISPR)-associated (Cas) effector protein" is a protein or polypeptide or domain thereof of the Type V CRISPR-Cas system that cleaves, cuts, or nicks a nucleic acid, binds a nucleic acid (e.g., a target nucleic acid and/or a guide nucleic acid), and/or that identifies, recognizes, or binds a guide nucleic acid as defined herein. In some embodiments, a Type V CRISPR-Cas effector protein may be an enzyme (e.g., a nuclease, endonuclease, nickase, etc.) or portion thereof and/or may function as an enzyme. In some embodiments, a Type V CRISPR-Cas effector protein refers to a Type V CRISPR-Cas nuclease polypeptide or domain that comprises nuclease activity or in which the nuclease activity has been reduced or eliminated, and/or comprises nickase activity or in which the nickase has been reduced or eliminated, and/or comprises single stranded DNA cleavage activity (ss DNAse activity) or in which the ss DNAse activity has been reduced or eliminated, and/or comprises self-processing RNAse activity or in which the self-processing RNAse activity has been reduced or eliminated. A Type V CRISPR-Cas effector protein may bind to a target nucleic acid. In some embodiments, a Type V CRISPR-Cas effector protein may be a Cas12 effector protein.

In some embodiments, a Type V CRISPR-Cas effector protein useful with the invention may comprise a mutation in its nuclease active site (e.g., RuvC, HNH, e.g., RuvC site of a Cas12a nuclease domain). A Type V CRISPR-Cas effector protein having a mutation in its nuclease active site, and therefore, no longer comprising nuclease activity, is commonly referred to as "dead," e.g., dCas12a. In some embodiments, a Type V CRISPR-Cas effector protein having a mutation in its nuclease active site may have impaired activity or reduced activity as compared to the same Type V CRISPR-Cas effector protein without the mutation, e.g., a nickase such as a Cas12a nickase.

A Type V CRISPR-Cas effector protein useful with embodiments of the invention may be any Type V CRISPR-Cas nuclease. A Type V CRISPR-Cas nuclease useful with this invention as an effector protein can include, but is not limited, to Cas12a (Cpfl), Cas12b, Cas12c (C2c3), Cas12d (CasY), Cas12e (CasX), Cas12g, Cas12h, Cas12i, C2c1, C2c4, C2c5, C2c8, C2c9, C2c10, Cas14a, Cas14b, and/or Cas14c nuclease. In some embodiments, a Type V CRISPR-Cas nuclease polypeptide or domain useful with embodiments of the invention may be a Cas12a polypeptide or domain. In some embodiments, a Type V CRISPR-Cas effector protein useful with embodiments of the invention may be a nickase, optionally, a Cas12a nickase.

In some embodiments, the Type V CRISPR-Cas effector protein may be a Cas12a effector protein. Cas12a differs in several respects from the more well-known Type II CRISPR Cas9. For example, Cas9 recognizes a G-rich protospacer-adjacent motif (PAM) that is 3' to its guide RNA (gRNA, sgRNA, crRNA, crDNA, CRISPR array) binding site (protospacer, target nucleic acid, target DNA) (3'-NGG), while Cas12a recognizes a T-rich PAM that is located 5' to the target nucleic acid (5'-TTN, 5'-TTTN. In fact, the orientations in which Cas9 and Cas12a bind their guide RNAs are very nearly reversed in relation to their N and C termini. Furthermore, Cas12a enzymes use a single guide RNA (gRNA, CRISPR array, crRNA) rather than the dual guide RNA (sgRNA (e.g., crRNA and tracrRNA)) found in natural Cas9 systems, and Cas12a processes its own gRNAs. Additionally, Cas12a nuclease activity produces staggered DNA double stranded breaks instead of blunt ends produced by Cas9 nuclease activity, and Cas12a relies on a single RuvC domain to cleave both DNA strands, whereas Cas9 utilizes an HNH domain and a RuvC domain for cleavage.

A Type V CRISPR-Cas effector protein may be a CRISPR-Cas12a polypeptide or CRISPR-Cas12a domain obtained from any known or later identified Cas12a (previously known as Cpfl) (see, e.g., U.S. Patent No. 9,790,490, which is incorporated by reference for its disclosures of Cpf1 (Cas12a) sequences). The term "Cas12a", "Cas12a polypeptide" or "Cas12a domain" refers to an RNA-guided polypeptide comprising a Cas12a polypeptide, or a fragment thereof, which comprises the guide nucleic acid binding domain of Cas12a and/or an active, inactive, or partially active DNA cleavage domain of Cas12a and/or the RNA-guided polypeptide may be have nuclease activity. In some embodiments, a Cas12a useful with the invention may comprise a mutation in the nuclease active site (e.g., RuvC site of the Cas12a domain). A Cas12a domain or Cas12a polypeptide having a mutation in its nuclease active site, and therefore, no longer comprising nuclease activity, is commonly referred to as deadCas12a (e.g., dCas12a). In some embodiments, a Cas12a domain or Cas12a polypeptide having a mutation in its nuclease active site may have impaired activity (e.g., may have impaired nickase activity).

In some embodiments, a Type V CRISPR-Cas effector protein (e.g., a Cas12a polypeptide) may be optimized for expression in an organism, for example, in an animal, a plant, a fungus, an archaeon, or a bacterium. In some embodiments, a Type V CRISPR-Cas effector protein(e.g., Cas12a polypeptide) may be optimized for expression in a plant.

Any deaminase or domain or polypeptide thereof useful for base editing may be used with this invention. A "cytosine deaminase" and "cytidine deaminase" as used herein refer to a polypeptide or domain thereof that catalyzes or is capable of catalyzing cytosine deamination in that the polypeptide or domain catalyzes or is capable of catalyzing the removal of an amine group from a cytosine base. Thus, a cytosine deaminase may result in conversion of cystosine to a thymidine (through a uracil intermediate), causing a C to T conversion, or a G to A conversion in the complementary strand in the genome. Thus, in some embodiments, the cytosine deaminase encoded by the polynucleotide of the invention generates a C→T conversion in the sense (e.g., "+"; template) strand of the target nucleic acid or a G →A conversion in antisense (e.g., "-", complementary) strand of the target nucleic acid. In some embodiments, a cytosine deaminase encoded by a polynucleotide of the invention generates a C to T, G, or A conversion in the complementary strand in the genome.

A cytosine deaminase useful with this invention may be any known or later identified cytosine deaminase from any organism (see, e.g., U.S. Patent No. 10,167,457 and Thuronyi et al. Nat. Biotechnol. 37:1070-1079 (2019), each of which is incorporated by reference herein for its disclosure of cytosine deaminases). Cytosine deaminases can catalyze the hydrolytic deamination of cytidine or deoxycytidine to uridine or deoxyuridine, respectively. Thus, in some embodiments, a deaminase or deaminase domain useful with this invention may be a cytidine deaminase domain that can catalyze the hydrolytic deamination of cytosine to uracil. In some embodiments, a cytosine deaminase may be a variant of a naturally-occurring cytosine deaminase, including but not limited to a primate (e.g., a human, monkey, chimpanzee, gorilla), a dog, a cow, a rat or a mouse. Thus, in some embodiments, an cytosine deaminase useful with the invention may be about 70% to about 100% identical to a wild type cytosine deaminase (e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identical, and any range or value therein, to a naturally occurring cytosine deaminase).

In some embodiments, a cytosine deaminase useful with the invention may be an apolipoprotein B mRNA-editing complex (APOBEC) family deaminase. In some embodiments, the cytosine deaminase may be an APOBEC1 deaminase, an APOBEC2 deaminase, an APOBEC3A deaminase, an APOBEC3B deaminase, an APOBEC3C deaminase, an APOBEC3D deaminase, an APOBEC3F deaminase, an APOBEC3G deaminase, an APOBEC3H deaminase, an APOBEC4 deaminase, a human activation induced deaminase (hAID), an rAPOBEC1, FERNY, and/or a CDA1, optionally a pmCDA1, an atCDA1 (e.g., At2g19570), and evolved versions of the same. Evolved deaminases are disclosed in, for example, U.S. Patent No. 10,113,163, Gaudelli et al. Nature 551(7681):464-471 (2017)) and Thuronyi et al. (Nature Biotechnology 37: 1070-1079 (2019)), each of which are incorporated by reference herein for their disclosure of deaminases and evolved deaminases. In some embodiments, a cytosine deaminase may be an APOBEC1 deaminase, optionally an APOBEC1 deaminase having the amino acid sequence of **SEQ ID NO:23.** In some embodiments, a cytosine deaminase may be an APOBEC3A deaminase, optionally an APOBEC3A deaminase having the amino acid sequence of **SEQ ID NO:24.** In some embodiments, a cytosine deaminase may be an CDA1 deaminase, optionally a CDA1 having the amino acid sequence of **SEQ ID NO:25.** In some embodiments, a cytosine deaminase may be a FERNY deaminase, optionally a FERNY having the amino acid sequence of **SEQ ID NO:26.** In some embodiments, the cytosine deaminase may be a rAPOBEC1 deaminase, optionally a rAPOBEC1 deaminase having the amino acid sequence of **SEQ ID NO:27.** In some embodiments, the cytosine deaminase may be a hAID deaminase, optionally a hAID having the amino acid sequence of **SEQ ID NO:28** or **SEQ ID NO:29.**

In some embodiments, a cytosine deaminase useful with the invention may be about 70% to about 100% identical (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or 100% identical) to the amino acid sequence of a naturally occurring cytosine deaminase (e.g., "evolved deaminases") (see, e.g., **SEQ ID NO:30, SEQ ID NO:31, SEQ ID NO:32).** In some embodiments, a cytosine deaminase useful with the invention may be about 70% to about 99.5% identical (e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.5% identical) to the amino acid sequence of any one of **SEQ ID NO:23-32** (e.g., at least 80%, at least 85%, at least 90%, at least 92%, at least 95%, at least 96%, at least 97%, at least 98%, at least 99%, or at least 99.5% identical to the amino acid sequence of **SEQ ID NO:23-32).** In some embodiments, a polynucleotide encoding a cytosine deaminase may be codon optimized for expression in an organism (e.g., a plant) and the codon optimized polypeptide may be about 70% to 99.5% identical to the reference polynucleotide.

An "adenine deaminase" and "adenosine deaminase" as used herein refer to a polypeptide or domain thereof that catalyzes or is capable of catalyzing the hydrolytic deamination (e.g., removal of an amine group from adenine) of adenine or adenosine. In some embodiments, an adenine deaminase may catalyze the hydrolytic deamination of adenosine or deoxyadenosine to inosine or deoxyinosine, respectively. In some embodiments, the adenosine deaminase may catalyze the hydrolytic deamination of adenine or adenosine in DNA. In some embodiments, an adenine deaminase encoded by a nucleic acid construct of the invention may generate an A→G conversion in the sense (e.g., "+"; template) strand of the target nucleic acid or a T→C conversion in the antisense (e.g., "-", complementary) strand of the target nucleic acid. An adenine deaminase useful with this invention may be any known or later identified adenine deaminase from any organism (see, e.g., U.S. Patent No. 10,113,163, which is incorporated by reference herein for its disclosure of adenine deaminases).

In some embodiments, an adenosine deaminase may be a variant of a naturally-occurring adenine deaminase. Thus, in some embodiments, an adenosine deaminase may be about 70% to 100% identical to a wild type adenine deaminase (e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identical, and any range or value therein, to a naturally occurring adenine deaminase). In some embodiments, the deaminase or deaminase does not occur in nature and may be referred to as an engineered, mutated or evolved adenosine deaminase. Thus, for example, an engineered, mutated or evolved adenine deaminase polypeptide or an adenine deaminase may be about 70% to 99.9% identical to a naturally occurring adenine deaminase polypeptide (e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8% or 99.9% identical, and any range or value therein, to a naturally occurring adenine deaminase polypeptide or adenine deaminase domain). In some embodiments, the adenosine deaminase may be from a bacterium, (e.g., *Escherichia coli*, *Staphylococcus aureus*, *Haemophilus influenzae*, *Caulobacter crescentus*, and the like). In some embodiments, a polynucleotide encoding an adenine deaminase polypeptide may be codon optimized for expression in a plant.

In some embodiments, an adenine deaminase may be a wild type tRNA-specific adenosine deaminase, e.g., a tRNA-specific adenosine deaminase (TadA) and/or a mutated/evolved adenosine deaminase, e.g., mutated/evolved tRNA-specific adenosine deaminase (TadA*). In some embodiments, TadA may be from *E*. *coli.* In some embodiments, the TadA may be modified, e.g., truncated, missing one or more N-terminal and/or C-terminal amino acids relative to a full-length TadA (e.g., 1, 2, 3, 4, 5, 6, 7, 8, 9, 10, 11, 12, 13, 14, 15, 6, 17, 18, 19, or 20 N-terminal and/or C terminal amino acid residues may be missing relative to a full length TadA. In some embodiments, a TadA polypeptide or TadA domain does not comprise an N-terminal methionine. In some embodiments, a wild type *E*. *coli* TadA comprises the amino acid sequence of **SEQ ID NO:33.** In some embodiments, a mutated/evolved *E*. *coli* TadA* comprises the amino acid sequence of **SEQ** ID **NOs:34-37** (e.g., **SEQ ID NOs: 34, 35, 36,** or **37).** In some embodiments, a polynucleotide encoding a TadA/TadA* may be codon optimized for expression in a plant. In some embodiments, an adenine deaminase may comprise all or a portion of an amino acid sequence of any one of **SEQ ID NOs:33-43.**

A "uracil glycosylase inhibitor" or "UGI" useful with the invention may be any protein or polypeptide or domain thereof that is capable of inhibiting a uracil-DNA glycosylase base-excision repair enzyme. In some embodiments, a UGI comprises a wild type UGI or a fragment thereof. In some embodiments, a UGI useful with the invention may be about 70% to about 100% identical (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% or 100% identical and any range or value therein) to an amino acid sequence of a naturally occurring UGI. In some embodiments, a UGI may comprise the amino acid sequence of **SEQ ID NO:44** or a polypeptide having about 70% to about 99.5% identity to the amino acid sequence of **SEQ ID NO:44** (e.g., at least 80%, at least 85%, at least 90%, at least 92%, at least 95%, at least 96%, at least 97%, at least 98%, at least 99%, or at least 99.5% identical to the amino acid sequence of **SEQ ID NO:44).** For example, in some embodiments, a UGI may comprise a fragment of the amino acid sequence of **SEQ ID NO:44** that is 100% identical to a portion of consecutive nucleotides (e.g., 10, 15, 20, 25, 30, 35, 40, 45, 50, 55, 60, 65, 70, 75, 80 consecutive nucleotides; e.g., about 10, 15, 20, 25, 30, 35, 40, 45, to about 50, 55, 60, 65, 70, 75, 80 consecutive nucleotides) of the amino acid sequence of **SEQ ID NO:44.** In some embodiments, a UGI may be a variant of a known UGI (e.g., **SEQ ID NO:44)** having about 70% to about 99.5% identity (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5% identity, and any range or value therein) to the known UGI. In some embodiments, a polynucleotide encoding a UGI may be codon optimized for expression in a plant (e.g., a plant) and the codon optimized polypeptide may be about 70% to about 99.5% identical to the reference polynucleotide.

In some embodiments, a nucleic acid construct of this invention comprising, for example, a Type V CRISPR-Cas fusion protein comprising a Type V CRISPR-Cas effector protein fused to a peptide tag, a deaminase fusion protein comprising a deaminase fused to an affinity polypeptide that binds to the peptide tag, and a guide nucleic acid; or a Type V CRISPR-Cas fusion protein comprising a Type V CRISPR-Cas effector protein, a recruiting guide nucleic acid comprising a guide nucleic acid linked to an RNA recruiting motif and a deaminase fusion protein comprising a deaminase fused to an affinity polypeptide that binds to the RNA recruiting motif may further comprise/encode a polypeptide of interest. In some embodiments, a polypeptide of interest may be a uracil glycosylase inhibitor (UGI) (e.g., uracil-DNA glycosylase inhibitor) polypeptide or domain, optionally wherein the UGI may be codon optimized for expression in an organism (e.g., a plant). In some embodiments, a glycosylase inhibitor may be fused to a Type V CRISPR-Cas effector protein and/or a deaminase. In some embodiments, a glycosylase inhibitor may be recruited to the Type V CRISPR-Cas effector protein and/or the deaminase via methods and constructs disclosed herein for protein-protein recruitment, protein-RNA recruitment, and/or chemical recruitment. Thus, as an example a glycosylase inhibitor may be recruited to the Type V CRISPR-Cas effector protein and/or deaminase utilizing a peptide tag/affinity polypeptide, an RNA recruiting motif/affinity polypeptide and/or biotin-streptavidin interaction (or other chemical interaction) as described herein.

The nucleic acid constructs of the invention comprising a Type V CRISPR-Cas effector protein or a fusion protein thereof may be used in combination with a guide nucleic acid (e.g., gRNA, CRISPR array, CRISPR RNA, crRNA) or recruiting guide nucleic acid that is designed to function with the encoded Type V CRISPR-Cas effector protein to modify a target nucleic acid. A guide nucleic acid and/or recruiting guide nucleic acid useful with this invention may comprise at least one spacer sequence and at least one repeat sequence. The guide nucleic acids and recruiting guide nucleic acids are capable of forming a complex with a Type V CRISPR-Cas effector protein of the present invention (e.g., a Type V CRISPR-Cas effector protein that is encoded and expressed by a nucleic acid construct of the invention) and the spacer sequence is capable of hybridizing to a target nucleic acid, thereby guiding the complex (e.g., the Type V CRISPR-Cas effector protein to the target nucleic acid), whereby the target nucleic acid may be modified (e.g., cleaved or edited) and/or modulated (e.g., modulating transcription) optionally by a deaminase (e.g., a cytosine deaminase and/or adenine deaminase that is optionally present in and/or recruited to the complex).

In some embodiments, a nucleic acid construct encoding a Type V CRISPR-Cas effector protein (e.g., Cas12a, Cas12b, Cas12c (C2c3), Cas12d (CasY), Cas12e (CasX), Cas12g, Cas12h, Cas12i, C2c1, C2c4, C2c5, C2c8, C2c9, C2c10, Cas14a, Cas14b, and/or Cas14c) fused to a peptide tag (e.g., a Type V CRISPR-Cas effector fusion protein), and a deaminase fusion protein comprising a deaminase fused to an affinity polypeptide that binds to the peptide tag may be used in combination with a Type V CRISPR-Cas guide nucleic acid to modify a target nucleic acid, wherein the guide nucleic acid binds to the target nucleic acid and guides the Type V CRISPR-Cas effector protein to the target nucleic acid, and the deaminase fusion protein is recruited to the Type V CRISPR-Cas effector protein and to the target nucleic acid via the binding of the affinity polypeptide of the deaminase to the peptide tag of the Type V CRISPR-Cas effector protein, whereby the deaminase of the deaminase fusion protein can then deaminate a cytosine base in the target nucleic acid, thereby modifying (e.g., editing) the target nucleic acid.

In some embodiments, a nucleic acid construct encoding a Type V CRISPR-Cas effector protein (e.g., Cas12a, Cas12b, Cas12c (C2c3), Cas12d (CasY), Cas12e (CasX), Cas12g, Cas12h, Cas12i, C2c1, C2c4, C2c5, C2c8, C2c9, C2c10, Cas14a, Cas14b, and/or Cas14c), and a deaminase fusion protein comprising a deaminase fused to an affinity polypeptide that binds to the RNA recruiting motif may be used in combination with a recruiting guide nucleic acid comprising a guide nucleic acid linked to an RNA recruiting motif to modify a target nucleic acid, wherein the recruiting guide nucleic acid binds to the target nucleic acid and guides the Type V CRISPR-Cas effector protein to the target nucleic acid, and the deaminase fusion protein is recruited to the target nucleic acid via the binding of the affinity polypeptide to the RNA recruiting motif of the recruiting guide nucleic acid, whereby the deaminase of the deaminase fusion protein can then deaminate a cytosine base in the target nucleic acid, thereby modifying (e.g., editing) the target nucleic acid.

A "guide nucleic acid," "guide RNA," "gRNA," "CRISPR RNA/DNA" "crRNA", or "crDNA" as used herein refers to a nucleic acid that comprises at least one spacer sequence, which is complementary to (and hybridizes to) a target DNA (e.g., protospacer), and at least one repeat sequence (e.g., a repeat of a Type V CRISPR-Cas system, or a fragment or portion thereof, including but not limited to, Cas12a, Cas12b, Cas12c (C2c3), Cas12d (CasY), Cas12e (CasX), Cas12g, Cas12h, Cas12i, C2c1, C2c4, C2c5, C2c8, C2c9, C2c10, Cas14a, Cas14b, and/or Cas14c, or a fragment thereof), wherein the repeat sequence may be linked to the 5' end and/or the 3' end of the spacer sequence. The design of a gRNA of this invention is based on a Type V CRISPR-Cas system. In some embodiments, the guide nucleic acid comprises DNA. In some embodiments, the guide nucleic acid comprises RNA. In some embodiments, a Type V CRISPR-Cas effector protein, e.g., a Cas12a gRNA, may comprise, from 5' to 3', a repeat sequence (e.g., a full length repeat sequence or portion thereof ("handle"); e.g., pseudoknot-like structure) and a spacer sequence.

A "recruiting guide nucleic acid" or "recruiting guide RNA" as used herein refer to a guide nucleic acid as defined herein that comprises an RNA recruiting motif. In some embodiments, the RNA recruiting motif may be linked to the 3' end or the 5' end of the recruiting guide nucleic acid. In some embodiments, the RNA recruiting motif may be inserted into the recruiting guide nucleic acid (e.g., within the hairpin loop). An RNA recruiting motif useful with this invention may be any RNA motif capable of being recognized by an affinity polypeptide, e.g., the RNA recruiting motif is capable of being bound by the affinity polypeptide. An RNA recruiting motif and its corresponding affinity polypeptide may include, but is not limited to, a telomerase Ku binding motif (e.g., Ku binding hairpin) and an affinity polypeptide of Ku (e.g., Ku heterodimer); a telomerase Sm7 binding motif and an affinity polypeptide of Sm7; an MS2 phage operator stem-loop and an affinity polypeptide of MS2 Coat Protein (MCP), a PP7 phage operator stem-loop and an affinity polypeptide of PP7 Coat Protein (PCP); an SfMu phage Com stem-loop and an affinity polypeptide of Com RNA binding protein; a PUF binding site (PBS) and a corresponding Pumilio/fem-3 mRNA binding factor (PUF); and/or a synthetic RNA-aptamer and a corresponding aptamer ligand. See, for example, WO2018/129129 and U.S. Patent Application Publication Nos. 20190218261 and 20180094257, each of which is incorporated by reference herein for their disclosure of RNA recruiting motifs.

In some embodiments, a recruiting guide nucleic acid may be linked to one RNA recruiting motif or to two or more RNA recruiting motifs (e.g., about 2, 3, 4, 5, 6, 7, 8, 9, 10 or more copies of an RNA recruiting motif; e.g., about 2 to about 5, about 2 to about 8, about 2 to about 10, about 3 to about 5, about 3 to about 8, about 5 to about 8, about 5 to about 10, and the like, recruiting motifs), optionally wherein the two or more RNA recruiting motifs may be the same RNA recruiting motif or may be different RNA recruiting motifs. Exemplary RNA recruiting motifs and corresponding affinity polypeptides that may be useful with this invention can include, but are not limited to, **SEQ ID NOs:45-55.**

In some embodiments, a guide nucleic acid and/or recruiting guide nucleic acid may comprise more than one repeat sequence-spacer sequence (e.g., 2, 3, 4, 5, 6, 7, 8, 9, 10, or more repeat-spacer sequences) (e.g., repeat-spacer-repeat, e.g., repeat-spacer-repeat-spacer-repeat-spacer-repeat-spacer-repeat-spacer, and the like). The guide nucleic acids or recruiting guide nucleic acids of this invention are synthetic, human-made and not found in nature. A gRNA can be quite long and may be used as an aptamer (like in the MS2 recruitment strategy) or other RNA structures hanging off the spacer, e.g., a recruiting guide nucleic acid.

A "repeat sequence" as used herein, refers to, for example, any repeat sequence of a wild-type Type V CRISPR Cas locus (e.g., Cas12a locus, Cas12b locus, Cas12c locus (C2c3), Cas12d locus (CasY), Cas12e locus (CasX), Cas12g locus, Cas12h locus, Cas12i locus, C2c1 locus, C2c4 locus, C2c5 locus, C2c8 locus, C2c9 locus, C2c10 locus, Cas14a locus, Cas14b locus, and/or Cas14c locus, or a fragment thereof) or a repeat sequence of a synthetic crRNA that is functional with a Type V CRISPR-Cas effector protein encoded by the nucleic acid constructs of the invention. A repeat sequence useful with this invention can be any known or later identified repeat sequence of a Type V CRISPR-Cas locus or it can be a synthetic repeat designed to function in a Type V CRISPR-Cas system. A repeat sequence may comprise a hairpin structure and/or a stem loop structure. In some embodiments, a repeat sequence may form a pseudoknot-like structure at its 5' end (i.e., "handle"). Thus, in some embodiments, a repeat sequence can be identical to or substantially identical to a repeat sequence from wild-type Type V CRISPR-Cas loci. A repeat sequence from a wild-type CRISPR-Cas locus may be determined through established algorithms, such as using the CRISPRfinder offered through CRISPRdb (see, Grissa et al. Nucleic Acids Res. 35(Web Server issue):W52-7). In some embodiments, a repeat sequence, or portion thereof, may be linked at its 3' end to the 5' end of a spacer sequence, thereby forming a repeat-spacer sequence (e.g., guide RNA, crRNA).

In some embodiments, a repeat sequence comprises, consists essentially of, or consists of at least 10 nucleotides depending on the particular repeat and whether the guide nucleic acid comprising the repeat is processed or unprocessed (e.g., about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 to 100 or more nucleotides, or any range or value therein; e.g., about). In some embodiments, a repeat sequence comprises, consists essentially of, or consists of about 10 to about 20, about 10 to about 30, about 10 to about 45, about 10 to about 50, about 10 to about 100, about 15 to about 30, about 15 to about 40, about 15 to about 45, about 15 to about 50, about 50 to about 100, about 20 to about 30, about 20 to about 40, about 20 to about 50, about 20 to about 100, about 30 to about 40, about 30 to about 50, about 30 to about 100, about 40 to about 80, about 40 to about 100, about 50 to about 100 or more nucleotides.

A repeat sequence linked to the 5' end of a spacer sequence can comprise a portion of a repeat sequence (e.g., 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more contiguous nucleotides of a wild type repeat sequence). In some embodiments, a portion of a repeat sequence linked to the 5' end of a spacer sequence can be about five to about ten consecutive nucleotides in length (e.g., about 5, 6, 7, 8, 9, 10 nucleotides) and have at least 90% sequence identity (e.g., at least about 90%, 91%, 92%, 93%, 94%, 95%, 96%, 97%, 98%, 99%, or more) to the same region (e.g., 5' end) of a wild type CRISPR Cas repeat nucleotide sequence. In some embodiments, a portion of a repeat sequence may comprise a pseudoknot-like structure at its 5' end (e.g., "handle").

A "spacer sequence" as used herein is a nucleotide sequence that is complementary to a target nucleic acid (e.g., target DNA) (e.g, protospacer). The spacer sequence can be fully complementary or substantially complementary (e.g., at least about 70% complementary (e.g., about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more)) to a target nucleic acid. Thus, in some embodiments, the spacer sequence can have one, two, three, four, or five mismatches as compared to the target nucleic acid, which mismatches can be contiguous or noncontiguous. In some embodiments, the spacer sequence can have 70% complementarity to a target nucleic acid. In other embodiments, the spacer nucleotide sequence can have 80% complementarity to a target nucleic acid. In still other embodiments, the spacer nucleotide sequence can have 85%, 90%, 95%, 96%, 97%, 98%, 99% or 99.5% complementarity, and the like, to the target nucleic acid (protospacer). In some embodiments, the spacer sequence is 100% complementary to the target nucleic acid. A spacer sequence may have a length from about 15 nucleotides to about 30 nucleotides (e.g., 15, 16, 17, 18, 19, 20, 21, 22, 23, 24, 25, 26, 27, 28, 29, or 30 nucleotides, or any range or value therein). Thus, in some embodiments, a spacer sequence may have complete complementarity or substantial complementarity over a region of a target nucleic acid (e.g., protospacer) that is at least about 15 nucleotides to about 30 nucleotides in length. In some embodiments, the spacer is about 20 nucleotides in length. In some embodiments, the spacer is about 21, 22, or 23 nucleotides in length.

In some embodiments, the 5' region of a spacer sequence of a guide nucleic acid may be identical to a target DNA, while the 3' region of the spacer may be substantially complementary to the target DNA (e.g., Type V CRISPR-Cas) or the 3' region of a spacer sequence of a guide nucleic acid may be identical to a target DNA, and therefore, the overall complementarity of the spacer sequence to the target DNA may be less than 100%. Thus, for example, in a guide for a Type V CRISPR-Cas system, the first 1, 2, 3, 4, 5, 6, 7, 8, 9, or 10 nucleotides in the 5' region (i.e., seed region) of, for example, a 20 nucleotide spacer sequence may be 100% complementary to the target DNA, while the remaining nucleotides in the 3' region of the spacer sequence are substantially complementary (e.g., at least about 70% complementary) to the target DNA. In some embodiments, the first 1 to 8 nucleotides (e.g., the first 1, 2, 3, 4, 5, 6, 7, 8, nucleotides, and any range therein) of the 5' end of the spacer sequence may be 100% complementary to the target DNA, while the remaining nucleotides in the 3' region of the spacer sequence are substantially complementary (e.g., at least about 50% complementary (e.g., 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more)) to the target DNA. A recruiting guide nucleic acid further comprises one or more recruiting motifs as described herein, which may be linked to the 5' end of the guide nucleic acid, the 3' end of the guide nucleic acid or the one or more RNA recruiting motif(s) may be inserted into the recruiting guide nucleic acid (e.g., within the hairpin loop).

In some embodiments, a seed region of a spacer may be about 8 to about 10 nucleotides in length, about 5 to about 6 nucleotides in length, or about 6 nucleotides in length.

As used herein, a "target nucleic acid", "target DNA," "target nucleotide sequence," "target region," or "target region in the genome" refer to a region of an organism's genome that is fully complementary (100% complementary) or substantially complementary (e.g., at least 70% complementary (e.g., 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more)) to a spacer sequence in a guide nucleic acid of this invention. A target region useful for a Type V CRISPR-Cas system, known as the protospacer adjacent motif (PAM), may be located adjacent to the spacer (or target) sequence. These PAM DNA sequences are typically described by referencing their sequence and location with respect to the non-target strand of the CRISPR complex. These PAM sequences can be either 3' (e.g., Type V CRISPR-Cas system) or 5' (e.g., Type II CRISPR-Cas system) to the ends of a protospacer sequence. A target region (also referred to as the protospacer) may be selected from any region of at least 15 consecutive nucleotides (e.g., 16, 17, 18, 19, 20, 21, 22, 23, 24, 25, 26, 27, 28, 29, 30 or more nucleotides, and the like) located immediately adjacent to a PAM sequence.

A "protospacer sequence" refers to the target double stranded DNA and specifically to the portion of the target DNA (e.g., or target region in the genome) that is fully or substantially complementary (and hybridizes) to the spacer sequence of the CRISPR repeat-spacer sequences (e.g., guide nucleic acids, CRISPR arrays, crRNAs).

In the case of Type V CRISPR-Cas (e.g., Cas12a) systems, the protospacer sequence is flanked (e.g., immediately adjacent to) by a protospacer adjacent motif (PAM). For Type V CRISPR-Cas systems, the PAM is located at the 5' end on the non-target strand and at the 3' end of the target strand (see below, as an example). 5'-NNNNNNNNNNNNNNNNNNN-3' RNA Spacer **(SEQ ID NO:56)** | | | | | | | | | | | | | | | | | | | | 3'**AAAN**NNNNNNNNNNNNNNNNNN-5' Target strand **(SEQ ID NO:57)** | | | | 5'**TTTN**NNNNNNNNNNNNNNNNNN-3' Non-target strand **(SEQ ID NO:58)** Guide structures and PAMs are described in by R. Barrangou (Genome Biol. 16:247 (2015)).

Canonical Type V CRISPR-Cas12a PAMs are T rich. In some embodiments, a canonical Cas12a PAM sequence may be 5'-TTN, 5'-TTTN, or 5'-TTTV. In some embodiments, non-canonical PAMs may be used but may be less efficient.

Additional PAM sequences may be determined by those skilled in the art through established experimental and computational approaches. Thus, for example, experimental approaches include targeting a sequence flanked by all possible nucleotide sequences and identifying sequence members that do not undergo targeting, such as through the transformation of target plasmid DNA (Esvelt et al. 2013. Nat. Methods 10:1116-1121; Jiang et al. 2013. Nat. Biotechnol. 31:233-239). In some aspects, a computational approach can include performing BLAST searches of natural spacers to identify the original target DNA sequences in bacteriophages or plasmids and aligning these sequences to determine conserved sequences adjacent to the target sequence (Briner and Barrangou. 2014. Appl. Environ. Microbiol. 80:994-1001; Mojica et al. 2009. Microbiology 155:733-740).

As described herein, a "peptide tag" may be employed to recruit one or more polypeptides. A peptide tag may be any polypeptide that is capable of being bound by a corresponding affinity polypeptide. A peptide tag may also be referred to as an "epitope" and when provided in multiple copies, a "multimerized epitope." Example peptide tags can include, but are not limited to, a GCN4 peptide tag (e.g., Sun-Tag), a c-Myc affinity tag, an HA affinity tag, a His affinity tag, an S affinity tag, a methionine-His affinity tag, an RGD-His affinity tag, a FLAG octapeptide, a strep tag or strep tag II, a V5 tag, and/or a VSV-G epitope. In some embodiments, a peptide tag may also include phosphorylated tyrosines in specific sequence contexts recognized by SH2 domains, characteristic consensus sequences containing phosphoserines recognized by 14-3-3 proteins, proline rich peptide motifs recognized by SH3 domains, PDZ protein interaction domains or the PDZ signal sequences, and an AGO hook motif from plants. Peptide tags are disclosed in WO2018/136783 and U.S. Patent Application Publication No. 2017/0219596, which are incorporated by reference for their disclosures of peptide tags. Peptide tags that may be useful with this invention can include, but are not limited to, **SEQ ID NO:59** and **SEQ ID NO:60.** An affinity polypeptide useful with a peptide tag includes, but is not limited to, **SEQ ID NO:61.**

Any epitope that may be linked to a polypeptide and for which there is a corresponding affinity polypeptide that may be linked to another polypeptide may be used with this invention as a peptide tag. In some embodiments, a peptide tag may comprise 1 or 2 or more copies of a peptide tag (e.g., peptide repeat unit, multimerized epitope (e.g., tandem repeats)) (e.g., 1, 2, 3, 4, 5, 6, 7, 8, 9, 10, 11, 12, 13, 14, 15, 16, 17, 18, 19, 20, 21, 22, 23, 24, 25 or more peptide tag(s)). In some embodiments, an affinity polypeptide that interacts with/binds to a peptide tag may be an antibody. In some embodiments, the antibody may be a scFv antibody. In some embodiments, an affinity polypeptide that binds to a peptide tag may be synthetic (e.g., evolved for affinity interaction) including, but not limited to, an affibody, an anticalin, a monobody and/or a DARPin (see, e.g., Sha et al., Protein Sci. 26(5):910-924 (2017)); Gilbreth (Curr Opin Struc Biol 22(4):413-420 (2013)), U.S. Patent No. 9,982,053, each of which are incorporated by reference in their entireties for the teachings relevant to affibodies, anticalins, monobodies and/or DARPins.

In some embodiments, a guide nucleic acid may be linked to an RNA recruiting motif, and a polypeptide to be recruited (e.g., a deaminase) may be fused to an affinity polypeptide that binds to the RNA recruiting motif, wherein the guide nucleic acid binds to the target nucleic acid and the RNA recruiting motif binds to the affinity polypeptide, thereby recruiting the polypeptide to the guide nucleic acid and contacting the target nucleic acid with the polypeptide (e.g., deaminase). In some embodiments, two or more polypeptides may be recruited to a guide nucleic acid, thereby contacting the target nucleic acid with two or more polypeptides (e.g., deaminases).

In some embodiments, the components for recruiting polypeptides and nucleic acids may include those that function through chemical interactions that may include, but are not limited to, rapamycin-inducible dimerization of FRB - FKBP; Biotin-streptavidin; SNAP tag; Halo tag; CLIP tag; DmrA-DmrC heterodimer induced by a compound; and/or a bifunctional ligand (e.g., fusion of two protein-binding chemicals together; e.g. dihyrofolate reductase (DHFR).

A peptide tag may comprise or be present in one copy or in 2 or more copies of the peptide tag (e.g., multimerized peptide tag or multimerized epitope) (e.g., about 2, 3, 4, 5, 6, 7, 8, 9, 10, 11, 12, 13, 14, 15, 16, 17, 18, 9, 20, 21, 22, 23, 24, or 25 or more peptide tags). When multimerized, the peptide tags may be fused directly to one another or they may be linked to one another via one or more amino acids (e.g., 1, 2, 3, 4, 5, 6, 7, 8, 9, 10, 11, 12, 13, 14, 15, 16, 17, 18, 19, 20 or more amino acids, optionally about 3 to about 10, about 4 to about 10, about 5 to about 10, about 5 to about 15, or about 5 to about 20 amino acids, and the like, and any value or range therein. Thus, in some embodiments, a Type V CRISPR-Cas fusion protein of the invention may comprise a Type V CRISPR-Cas effector protein fused to one peptide tag or to two or more peptide tags, optionally wherein the two or more peptide tags are fused to one another via one or more amino acid residues. In some embodiments, a peptide tag useful with the invention may be a single copy of a GCN4 peptide tag or epitope or may be a multimerized GCN4 epitope comprising about 2 to about 25 or more copies of the peptide tag (e.g., about 2, 3, 4, 5, 6, 7, 8, 9, 10, 11, 12, 13, 14, 15, 16, 17, 18, 19, 20, 21, 22, 23, 24, 25 or more copies of a GCN4 epitope or any range therein).

In some embodiments, a peptide tag may be fused to a Type V CRISPR-Cas protein. In some embodiments, a peptide tag may be fused or linked to the C-terminus of a Type V CRISPR-Cas effector protein to form a Type V CRISPR-Cas fusion protein. In some embodiments, a peptide tag may be fused or linked to the N-terminus of a Type V CRISPR-Cas effector protein to form a Type V CRISPR-Cas fusion protein.

In some embodiments, when a peptide tag comprises more than one peptide tag, the quantity and/or spacing of an epitope may be optimized within the peptide tag to maximize occupation of the peptide tags and minimize steric interference of, for example, deaminase domains, with each other.

An "affinity polypeptide" (e.g., "recruiting polypeptide") refers to any polypeptide that is capable of binding to its corresponding peptide tag or RNA recruiting motif. An affinity polypeptide for a peptide tag may be, for example, an antibody and/or a single chain antibody that specifically binds the peptide tag. In some embodiments, an antibody for a peptide tag may be, but is not limited to, an scFv antibody. In some embodiments, an affinity polypeptide may be fused or linked to the N-terminus of a deaminase (e.g., a cytosine deaminase or an adenine deaminase), optionally to recruit the deaminase to a recruiting guide nucleic acid or Type V CRISPR-Cas effector protein. In some embodiments, the affinity polypeptide is stable under the reducing conditions of a cell or cellular extract.

The nucleic acid constructs of the invention and/ guide nucleic acids and/or recruiting guide nucleic acids may be comprised in one or more expression cassettes as described herein. In some embodiments, a nucleic acid construct of the invention may be comprised in the same or in a separate expression cassette or vector from that comprising a guide nucleic acid and/or a recruiting guide nucleic acid.

When used in combination with guide nucleic acids and recruiting guide nucleic acids, the nucleic acid constructs of the invention (and expression cassettes and vectors comprising the same) may be used to modify a target nucleic acid and/or its expression. A target nucleic acid may be contacted with a nucleic acid construct of the invention and/or expression cassettes and/or vectors comprising the same prior to, concurrently with or after contacting the target nucleic acid with the guide nucleic acid/recruiting guide nucleic acid (and/or expression cassettes and vectors comprising the same.

The present invention further provides a method for modifying a target nucleic acid using a composition, complex (e.g., a ribonucleocomplex), system, nucleic acid construct, expression cassette and/or vector of the present invention. The methods may be carried out in an *in vivo* system (e.g., in a cell or in an organism) or in an *in vitro* system (e.g., cell free). In some embodiments, the invention provides a method of modifying a target nucleic acid, the method comprising contacting the target nucleic acid with: (a) a Type V CRISPR-Cas effector protein; (b) a deaminase, optionally wherein the target nucleic acid is contacted with two or more deaminases; and(c) a guide nucleic acid, wherein the deaminase is recruited to the Type V CRISPR-Cas effector protein (e.g., recruited via a protein to protein interaction, RNA to protein interaction, and/or chemical interaction), optionally wherein the Type V CRISPR-Cas effector protein, the deaminase and guide nucleic acid are co-expressed, thereby modifying the target nucleic acid.

In some embodiments, a method of modifying a target nucleic acid is provided, the method comprising: contacting the target nucleic acid with: (a) a Type V CRISPR-Cas fusion protein comprising a Type V CRISPR-Cas effector protein fused to a peptide tag (e.g., an epitope or a multimerized epitope); (b) a deaminase fusion protein comprising a deaminase fused to an affinity polypeptide that binds to the peptide tag, optionally wherein the target nucleic acid is contacted with two or more deaminase fusion proteins; and (c) a guide nucleic acid, optionally wherein the Type V CRISPR-Cas fusion protein, the deaminase fusion protein and guide nucleic acid are co-expressed, thereby modifying the target nucleic acid. In some embodiments, the peptide tag may be one copy of a peptide tag, or two or more copies (e.g., two or more epitopes) of a peptide tag as described herein. In some embodiments, the peptide tag may be, for example, a GCN4 peptide tag (e.g., Sun-Tag) comprising one to about twenty five repeat units. In some embodiments, the affinity polypeptide may be an antibody. In some embodiments, multiple deaminases may be contacted with the target nucleic acid and recruited by the Type V CRISPR-Case fusion protein. In some embodiments, the target nucleic acid may be contacted with more than one guide nucleic acid, which may comprise the same or different spacer and/or repeat as one another, thereby allowing the targeting of different sites on the target nucleic acid and/or interacting with different Type V CRISPR-Cas effector proteins (e.g., Cas12a, Cas12b, Cas12c (C2c3), Cas12d (CasY), Cas12e (CasX), Cas12g, Cas12h, Cas12i, C2c1, C2c4, C2c5, C2c8, C2c9, C2c10, Cas14a, Cas14b, and/or Cas14c).

In some embodiments, the invention provides a method of modifying a target nucleic acid, the method comprising contacting the target nucleic acid with: (a) a Type V CRISPR-Cas effector protein; (b) a recruiting guide nucleic acid comprising a guide RNA linked to an RNA recruiting motif, and (c) a deaminase fusion protein comprising a deaminase fused to an affinity polypeptide that binds to the RNA recruiting motif, optionally wherein the target nucleic acid may be contacted with two or more deaminase fusion proteins, wherein the Type V CRISPR-Cas effector protein, the deaminase fusion protein and the recruiting guide nucleic acid are co-expressed, thereby modifying the target nucleic acid. Any RNA recruiting motif(s) as described herein may be used with the methods of this invention. In some embodiments, multiple deaminases may be contacted with the target nucleic acid and may be recruited by the recruiting guide nucleic acid. In some embodiments, the target nucleic acid may be contacted with more than one recruiting guide nucleic acid, which may comprise the same or different RNA recruiting motif and/or spacer and/or repeat as one another, thereby allowing the targeting of different sites (e.g., 2, 3, 4, 5, or more different sites) on the target nucleic acid, allowing interaction with different Type V CRISPR-Cas effector proteins and recruiting of multiple polypeptides, which polypeptides may be the same or different.

In some embodiments, a deaminase useful for modifying a target nucleic acid may be a cytosine deaminase and/or an adenine deaminase as described herein. In some embodiments, the cytosine deaminase may be an apolipoprotein B mRNA editing catalytic polypeptide-like (APOBEC) domain, a human activation induced deaminase (hAID), a FERNY deaminase, and/or a CDA1 deaminase. In some embodiments, the APOBEC deaminase may be an APOBEC3A deaminase. In some embodiments, the adenine deaminase may be a TadA (tRNA-specific adenosine deaminase) and/or TadA* (evolved tRNA-specific adenosine deaminase). In some embodiments, the methods of the invention may further comprise introducing/expressing a glycosylase inhibitor and/or a polynucleotide encoding a glycosylase inhibitor (e.g., a uracil-DNA glycosylase inhibitor (UGI)), optionally wherein method may comprise introducing or expressing two or more glycosylase inhibitors. In some embodiments, the glycosylase inhibitor may be fused to the Type V CRISPR-Cas effector protein and/or the deaminase. In some embodiments, a glycosylase inhibitor may be recruited to a Type V CRISPR-Cas effector protein and/or a deaminase via methods and constructs disclosed herein for protein-protein recruitment, protein-RNA recruitment, and/or chemical recruitment. Thus, as an example a glycosylase inhibitor may be recruited to a Type V CRISPR-Cas effector protein and/or deaminase utilizing a peptide tag/affinity polypeptide, an RNA recruiting motif/affinity polypeptide and/or biotin-streptavidin interaction (or other chemical interaction) as described herein.

The methods of the invention may comprise contacting a target nucleic acid with CRISPR Cas effector proteins, deaminases, and/or fusion proteins thereof of the invention and/or polypeptides of interest, or the target nucleic acid may be contacted with polynucleotides encoding the CRISPR Cas effector proteins, deaminases, and fusion proteins thereof of the invention and/or polypeptides of interest, which polypeptides may be optionally comprised in one or more expression cassettes and/or vectors as described herein, said expression cassettes and/or vectors optionally comprising one or more guide nucleic acids/recruiting guide nucleic acids.

As described herein, the nucleic acids of the invention and/or expression cassettes and/or vectors comprising the same may be codon optimized for expression in an organism. An organism useful with this invention may be any organism or cell thereof for which nucleic acid modification may be useful. An organism can include, but is not limited to, any animal, any plant, any fungus, any archaeon, or any bacterium. In some embodiments, the organism may be a plant or cell thereof.

A target nucleic acid of any plant or plant part may be modified using the nucleic acid constructs of the invention. Any plant (or groupings of plants, for example, into a genus or higher order classification) may be modified using the nucleic acid constructs of this invention including an angiosperm, a gymnosperm, a monocot, a dicot, a C3, C4, CAM plant, a bryophyte, a fern and/or fern ally, a microalgae, and/or a macroalgae. A plant and/or plant part useful with this invention may be a plant and/or plant part of any plant species/variety/cultivar. The term "plant part," as used herein, includes but is not limited to, embryos, pollen, ovules, seeds, leaves, stems, shoots, flowers, branches, fruit, kernels, ears, cobs, husks, stalks, roots, root tips, anthers, plant cells including plant cells that are intact in plants and/or parts of plants, plant protoplasts, plant tissues, plant cell tissue cultures, plant calli, plant clumps, and the like. As used herein, "shoot" refers to the above ground parts including the leaves and stems. Further, as used herein, "plant cell" refers to a structural and physiological unit of the plant, which comprises a cell wall and also may refer to a protoplast. A plant cell can be in the form of an isolated single cell or can be a cultured cell or can be a part of a higher-organized unit such as, for example, a plant tissue or a plant organ.

Non-limiting examples of plants useful with the present invention include turf grasses (e.g., bluegrass, bentgrass, ryegrass, fescue), feather reed grass, tufted hair grass, miscanthus, arundo, switchgrass, vegetable crops, including artichokes, kohlrabi, arugula, leeks, asparagus, lettuce (*e.g.*, head, leaf, romaine), malanga, melons (*e.g.*, muskmelon, watermelon, crenshaw, honeydew, cantaloupe), cole crops (e.g., brussels sprouts, cabbage, cauliflower, broccoli, collards, kale, chinese cabbage, bok choy), cardoni, carrots, napa, okra, onions, celery, parsley, chick peas, parsnips, chicory, peppers, potatoes, cucurbits (e.g., marrow, cucumber, zucchini, squash, pumpkin, honeydew melon, watermelon, cantaloupe), radishes, dry bulb onions, rutabaga, eggplant, salsify, escarole, shallots, endive, garlic, spinach, green onions, squash, greens, beet (sugar beet and fodder beet), sweet potatoes, chard, horseradish, tomatoes, turnips, and spices; a fruit crop such as apples, apricots, cherries, nectarines, peaches, pears, plums, prunes, cherry, quince, fig, nuts (e.g., chestnuts, pecans, pistachios, hazelnuts, pistachios, peanuts, walnuts, macadamia nuts, almonds, and the like), citrus (e.g., clementine, kumquat, orange, grapefruit, tangerine, mandarin, lemon, lime, and the like), blueberries, black raspberries, boysenberries, cranberries, currants, gooseberries, loganberries, raspberries, strawberries, blackberries, grapes (wine and table), avocados, bananas, kiwi, persimmons, pomegranate, pineapple, tropical fruits, pomes, melon, mango, papaya, and lychee, a field crop plant such as clover, alfalfa, timothy, evening primrose, meadow foam, corn/maize (field, sweet, popcorn), hops, jojoba, buckwheat, safflower, quinoa, wheat, rice, barley, rye, millet, sorghum, oats, triticale, sorghum, tobacco, kapok, a leguminous plant (beans (e.g., green and dried), lentils, peas, soybeans), an oil plant (rape, canola, mustard, poppy, olive, sunflower, coconut, castor oil plant, cocoa bean, groundnut, oil palm), duckweed, *Arabidopsis*, a fiber plant (cotton, flax, hemp, jute), Cannabis (*e.g.*, *Cannabis sativa*, *Cannabis indica*, and *Cannabis ruderalis*), lauraceae (cinnamon, camphor), or a plant such as coffee, sugar cane, tea, and natural rubber plants; and/or a bedding plant such as a flowering plant, a cactus, a succulent and/or an ornamental plant (e.g., roses, tulips, violets), as well as trees such as forest trees (broad-leaved trees and evergreens, such as conifers; e.g., elm, ash, oak, maple, fir, spruce, cedar, pine, birch, cypress, eucalyptus, willow), as well as shrubs and other nursery stock. In some embodiments, the nucleic acid constructs of the invention and/or expression cassettes and/or vectors encoding the same may be used to modify maize, soybean, wheat, canola, rice, tomato, pepper, sunflower, raspberry, blackberry, black raspberry and/or cherry.

In some embodiments, the invention provides cells (e.g., plant cells, animal cells, bacterial cells, archaeon cells, and the like) comprising the polypeptides, polynucleotides, nucleic acid constructs, expression cassettes or vectors of the invention.

The present invention further comprises a kit or kits to carry out the methods of this invention. A kit of this invention can comprise reagents, buffers, and apparatus for mixing, measuring, sorting, labeling, etc, as well as instructions and the like as would be appropriate for modifying a target nucleic acid.

In some embodiments, the invention provides a kit for comprising one or more nucleic acid constructs of the invention, and/or expression cassettes and/or vectors and/or cells comprising the same as described herein, with optional instructions for the use thereof. In some embodiments, a kit may further comprise a CRISPR-Cas guide nucleic acid and/or recruiting guide nucleic acid (corresponding to the CRISPR-Cas effector protein encoded by the polynucleotide of the invention) and/or expression cassettes and/or vectors and or cells comprising the same. In some embodiments, a guide nucleic acid and/or recruiting guide nucleic acid may be provided on the same expression cassette and/or vector as one or more nucleic acid constructs of the invention. In some embodiments, the guide nucleic acid and/or recruiting guide nucleic acid may be provided on a separate expression cassette or vector from that comprising the one or more nucleic acid constructs of the invention.

Accordingly, in some embodiments, kits are provided comprising a nucleic acid construct comprising (a) a polynucleotide(s) as provided herein and (b) a promoter that drives expression of the polynucleotide(s) of (a). In some embodiments, the kit may further comprise a nucleic acid construct encoding a guide nucleic acid and/or recruiting guide nucleic acid, wherein the construct comprises a cloning site for cloning of a nucleic acid sequence identical or complementary to a target nucleic acid sequence into backbone of the guide nucleic acid and/or recruiting guide nucleic acid.

In some embodiments, the nucleic acid construct of the invention may be an mRNA that may encode one or more introns within the encoded polynucleotide(s). In some embodiments, the nucleic acid constructs of the invention, and/or an expression cassettes and/or vectors comprising the same, may further encode one or more selectable markers useful for identifying transformants (e.g., a nucleic acid encoding an antibiotic resistance gene, herbicide resistance gene, and the like).

The invention will now be described with reference to the following examples. It should be appreciated that these examples are not intended to limit the scope of the claims to the invention, but are rather intended to be exemplary of certain embodiments. Any variations in the exemplified methods that occur to the skilled artisan are intended to fall within the scope of the invention.

### EXAMPLES

### Example 1. SunTag-fused dCpf1 for C to T base editing

Eight copies of GCN4 epitopes were fused to the C-terminus of catalytically deactivated LbCpfl (dLbCpf1)(dLbCas12a) such that a sequence of **SEQ ID NO:62** was provided. In a separate plasmid, an antibody targeted toward the GCN4 epitope was fused to a variety of deaminases (scFv-deaminase) including rAPOBEC1, hAPOBEC3A, hAPOBEC3B, hAID, and pmCDA1 **(SEQ ID NOs:63-67).**

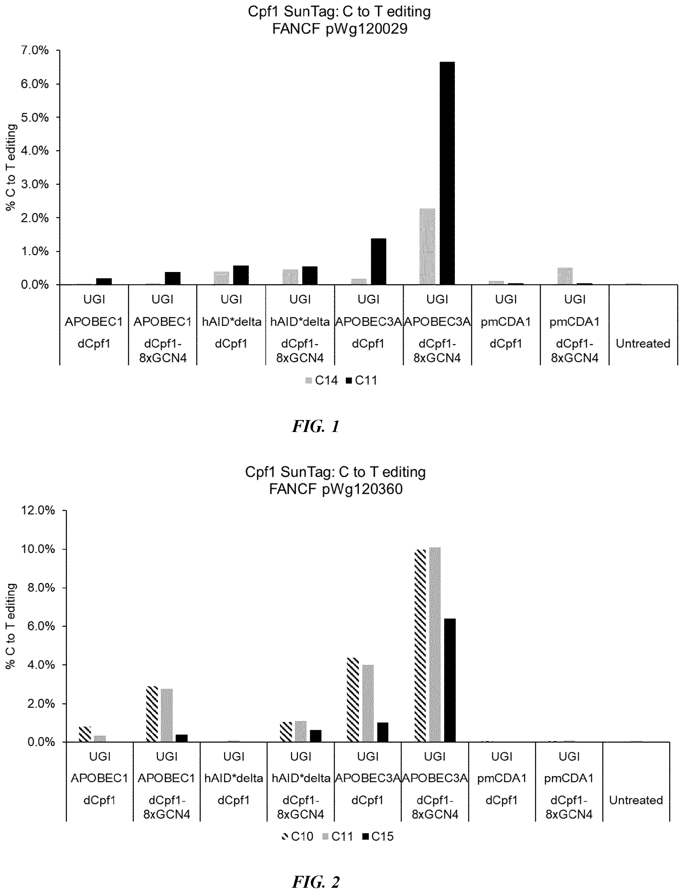

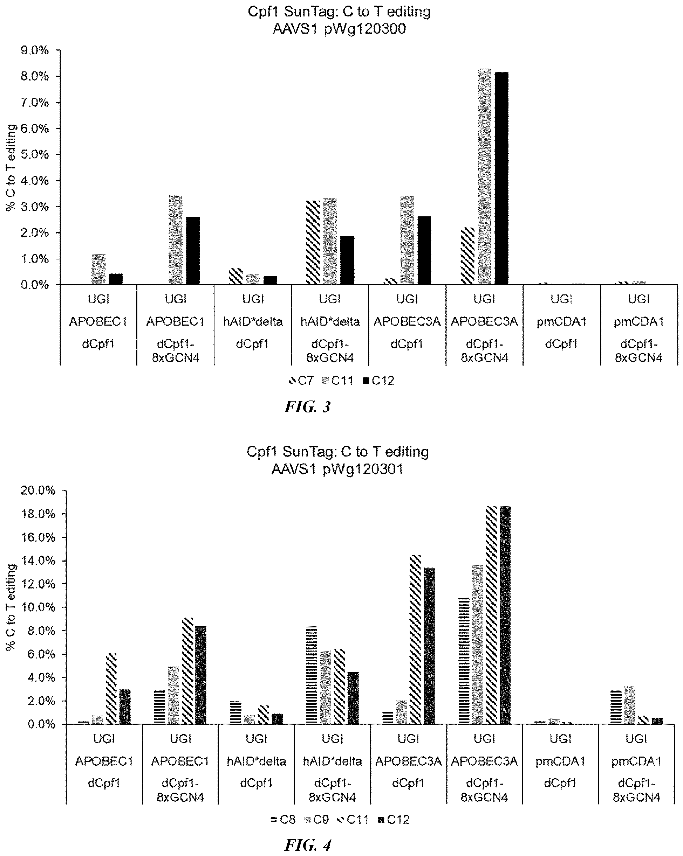

Plasmids encoding dLbCpf1-SunTag, scFv-deaminase, UGI, and a guide RNA were transfected in HEK293T cells. UGI was co-expressed to transiently suppress base excision repair during the base editing event, which improves efficiency and product purity (Nishida et al. Science 353(6305) (2016) (DOI: 10.1126/science.aaf8729)). A total of four different guide RNAs targeting endogenous genes were used to probe C to T base editing **(Table 1).** After three days, genome editing was quantified by next generation sequencing (NGS). While most deaminases tested showed varied and generally low C to T editing efficiencies, APOBEC3A showed consistently high C to T editing rates, ranging from ~7% to ~19% of treated cell population (not sorted for transfected cells) **(****Figs. 1-4****).** SunTag fusion to Cpfl is also functional in recruiting APOBEC3A since SunTag fusion provides up to 5 times higher editing efficiency compared to unfused dCpf1 **(****Figs. 1-4****).** Efficient editing is observed roughly within position 6-15 (position -4,-3,-2,-1 is designated as the PAM (TTTV)) **(****Figs. 1-4****).**

**Table 1: Targeted loci and corresponding spacer sequence.**

| Loci Name | Guide Name | Spacer Sequence |
|---|---|---|
| FANCF-002 | PWg120029 | ACCTTGGAGACGGCGACTCTCTG **(SEQ ID NO:68)** |
| FANCF-002 | PWg120360 | GCGGATGTTCCAATCAGTACGCA **(SEQ ID NO:69)** |
| AAVS1 | PWg120300 | TGTCACCAATCCTGTCCCTAGTG **(SEQ ID NO:70)** |
| AAVS1 | PWg120301 | TCTGTCCCCTCCACCCCACAGTG **(SEQ ID NO:71)** |

**Figs. 1-4** show that SunTag-fused dCpf1 can efficiently recruit deaminase domains that are co-expressed in the cell, and this can be used to effect significant levels of C to T base editing. This is the first demonstration of base editing using Cpfl without the use of the fusion architecture. Multiple additional components as described herein may be co-expressed either singly, expressed under single promoter or any combination.

### Example 2. SunTag-fused dCpf1 for A to G base editing

Eight copies of GCN4 epitopes were fused to the C-terminus of catalytically deactivated LbCpfl (dLbCpf1)(dLbCas12a) such that a sequence of **SEQ ID NO:62** was provided. In a separate plasmid, an antibody targeted toward the GCN4 epitope was fused to an evolved adenine deaminase (TadA8e) (scFv-deaminase) such that a sequence of **SEQ ID NO:72** was provided.

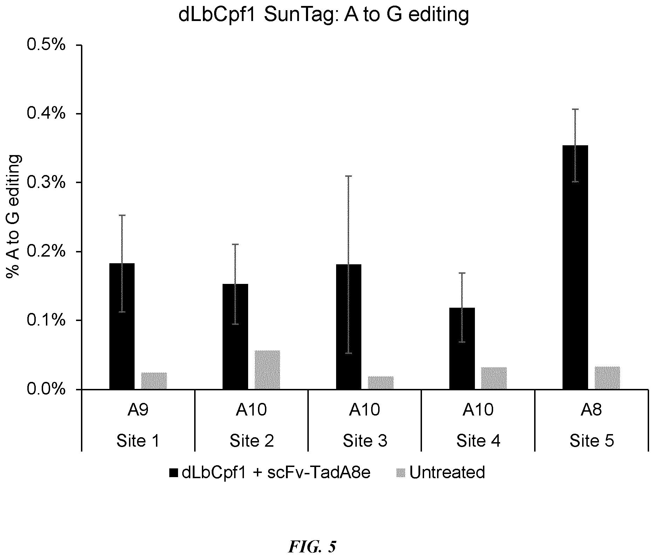

Plasmids encoding dLbCpf1-SunTag, scFv-TadA8e (Richter et al. Nat Biotechnol. 2020, 38(7):883-891) and a guide RNA were transfected in HEK293T cells. A total of five different guide RNAs including a spacer sequence of one of **SEQ ID NOs:73-77** were used to target endogenous genes (Sites 1-5) to probe A to G base editing **(****Fig. 5****).** After three days, genome editing is quantified by next generation sequencing (NGS).

Significant adenine deamination was observed at the expected targeting window around position 8-10 (position -4,-3,-2,-1 is designated as the PAM (TTTV)).

The constructs and methods of this invention are broadly applicable to many different types of systems including *in vitro* and i*n vivo* systems, and may utilize multiple different Type V CRISPR Cas effector proteins and multiple different deaminases, and in multiple types of organisms including animal (e.g., mammalian) and plant systems. By multiplexing guides, editing could be targeted toward multiple different loci within the genome at the same time.

### Example 3. SunTag-fused dCpf1 for C to T base editing in soybean plant

T-DNA vectors were constructed that contained expression cassettes for GCN4 epitope-tagged dCpf1 (LbCpfl and EnAsCpf1), a single-chain antibody-fused APOBEC3A (scFv-A3A), and a uracil glycosylase inhibitor (UGI). These components were either expressed via a single promoter, utilizing fusion and P2A linkers, or via multiple promoters driving expression of individual components **(Table 2).** Specifically, DaMV promoter was used to express the CRISPR-SunTag component, and Mt.Ubq2 promoter was used to express the deaminase and UGI components **(Table 2).** The TDNA vectors also included an antibiotic selection cassette and a guide RNA cassette, driven by a U6 promoter, including a sequence targeting either Target #1 or Target #2 in the target soybean gene. The T-DNA vectors were transformed into Agrobacteria and treated with soybean dried excised embryos to induce plant transformation. The leaves grown in antibiotic selection media were sampled around 4 weeks post-transformation. After extracting DNA, their genetic composition was analyzed using Illumina high-throughput amplicon sequencing.

Base editing activity (C to T change in the target spacer sequence) was detected in all the constructs tested at a rate varying from 20% to 88% **(Table 2).** Among the edited plants for each construct, the high-throughput sequencing analysis showed that each plant sample contained 0.77%-17.48% edited DNA per plant on average **(Table 2).** Editing was observed for both target genes tested (Target #1 and Target #2), and for both CRISPR enzymes tested (LbCpfl and EnAsCpf1) **(Table 2).** These results demonstrate that this approach for base editing is suitable for other target genes and other Type V CRISPR systems.

**Table 2: Expression constructs and their editing results.**

| **Expression constructs used** | **Target Gene** | **# plants assayed** | **# Edited plants** | **Global edit %** | **% Average editing per edited plant** | **% Standard Deviation per edited plant** |
|---|---|---|---|---|---|---|
| **DaMV promoter: dLbCas12a-8xGCN4, MtUbq2 promoter: scFv-A3A-UGI-GB1** | **#1** | **94** | **83** | **88%** | **6.91** | **11.77** |
| **DaMV promoter: dEnAsCas 12a-8xGCN4, MtUbq2 promoter: scFv-A3A-GB1-P2A-UGI** | **#1** | **94** | **41** | **44%** | **6.58** | **13.81** |
| **MtUbq2 promoter: dLbCas12a-8xGCN4-P2A-scFv-A3A-UGI-GB1** | **#1** | **94** | **30** | **32%** | **1.88** | **10.92** |
| **DaMV promoter: dEnAsCas 12a-8xGCN4, MtUbq2 promoter: scFv-A3A-UGI-GB1** | **#1** | **93** | **19** | **20%** | **0.77** | **0.79** |
| **DaMV promoter: dEnAsCas 12a-8xGCN4, MtUbq2 promoter: scFv-A3A-GB1, 35S promoter: UGI** | **#1** | **83** | **38** | **46%** | **4.56** | **5.99** |
| **DaMV promoter: dLbCas12a-8xGCN4, MtUbq2 promoter: scFv-A3A-UGI-GB1** | **#2** | **94** | **44** | **47%** | **8.53** | **11.21** |
| **DaMV promoter: dLbCas12a-8xGCN4, MtUbq2 promoter: scFv-A3A-GB1-P2A-UGI** | **#2** | **88** | **57** | **65%** | **17.48** | **21.14** |

The foregoing is illustrative of the present invention, and is not to be construed as limiting thereof. The invention is defined by the following claims, with equivalents of the claims to be included therein.

### SUMMARY PARAGRAPH:

1. A method of modifying a target nucleic acid, the method comprising:
   contacting the target nucleic acid with:
   (a) a Type V Clustered Regularly Interspaced Short Palindromic Repeats (CRISPR)-associated (Cas) (CRISPR-Cas) effector protein;
   (b) a deaminase, optionally wherein the target nucleic acid is contacted with two or more deaminases; and
   (c) a guide nucleic acid, wherein the deaminase is recruited to the Type V CRISPR-Cas effector protein (e.g., recruited via a protein to protein interaction, RNA to protein interaction, and/or chemical interaction), thereby modifying the target nucleic acid, optionally wherein the Type V CRISPR-Cas effector protein, the deaminase and guide nucleic acid are co-expressed.
2. A method of modifying a target nucleic acid, the method comprising:
   contacting the target nucleic acid with:
   (a) a Type V Clustered Regularly Interspaced Short Palindromic Repeats (CRISPR)-associated (Cas) (CRISPR-Cas) fusion protein comprising a Type V CRISPR-Cas effector protein fused to a peptide tag;
   (b) a deaminase fusion protein comprising a deaminase fused to an affinity polypeptide that binds to the peptide tag, optionally wherein the target nucleic acid is contacted with two or more deaminase fusion proteins; and
   (c) a guide nucleic acid, optionally wherein the Type V CRISPR-Cas fusion protein, the deaminase fusion protein and guide nucleic acid are co-expressed, thereby modifying the target nucleic acid.
3. A method of modifying a target nucleic acid, the method comprising:
   contacting the target nucleic acid with:
   (a) a Type V Clustered Regularly Interspaced Short Palindromic Repeats (CRISPR)-associated (Cas) (CRISPR-Cas) fusion protein comprising a Type V CRISPR-Cas effector protein fused to an affinity polypeptide that binds to a peptide tag;
   (b) a deaminase fusion protein comprising a deaminase fused to the peptide tag, optionally wherein the target nucleic acid is contacted with two or more deaminase fusion proteins; and
   (c) a guide nucleic acid, optionally wherein the Type V CRISPR-Cas fusion protein, the deaminase fusion protein and guide nucleic acid are co-expressed, thereby modifying the target nucleic acid.
4. A method of modifying a target nucleic acid, the method comprising:
   contacting the target nucleic acid with:
      (a) a Type V Clustered Regularly Interspaced Short Palindromic Repeats (CRISPR)-associated (Cas) (CRISPR-Cas) effector protein;
      (b) a recruiting guide nucleic acid comprising a guide RNA linked to an RNA recruiting motif, and
      (c) a deaminase fusion protein comprising a deaminase fused to an affinity polypeptide that binds to the RNA recruiting motif, optionally wherein the target nucleic acid is contacted with two or more deaminase fusion proteins; and
   optionally wherein the Type V CRISPR-Cas effector protein, the deaminase fusion protein and the recruiting guide nucleic acid are co-expressed, thereby modifying the target nucleic acid.
5. The method of paragraph 2 or paragraph 3, wherein the peptide tag comprises 2 or more copies of the peptide tag (e.g., two or more epitopes).
6. The method of any one of paragraphs 2, 3 or 5, wherein the peptide tag is a GCN4 peptide repeat unit (e.g., Sun-Tag), a c-Myc affinity tag, an HA affinity tag, a His affinity tag, an S affinity tag, a methionine-His affinity tag, an RGD-His affinity tag, a FLAG octapeptide, a strep tag or strep tag II, a V5 tag, and/or a VSV-G epitope.
7. The method of any one of paragraphs 2, 3, 5, or 6, wherein the affinity polypeptide is an antibody.
8. The method of paragraph 7, wherein the antibody is a scFv antibody.
9. The method of paragraph 4, wherein the recruiting guide nucleic acid is linked to two or more RNA recruiting motifs, optionally wherein the two or more RNA recruiting motifs are the same RNA recruiting motif or different RNA recruiting motifs.
10. The method of paragraph 4 or paragraph 9, wherein the RNA recruiting motif is a telomerase Ku binding motif (e.g., Ku binding hairpin) and the affinity polypeptide is a Ku polypeptide (e.g., Ku heterodimer); the RNA recruiting motif is a telomerase Sm7 binding motif and the affinity polypeptide is a Sm7 polypeptide; the RNA recruiting motif is an MS2 phage operator stem-loop and the affinity polypeptide is a MS2 Coat Protein (MCP); the RNA recruiting motif is a PP7 phage operator stem-loop and the affinity polypeptide is a PP7 Coat Protein (PCP); the RNA recruiting motif is an SfMu phage Com stem-loop and the affinity polypeptide is a Com RNA binding protein; the RNA recruiting motif is a Pumilio/fem-3 mRNA binding factor (PUF) and the affinity polypeptide is a PUF binding site (PBS) polypeptide; and/or the RNA recruiting motif is a synthetic RNA-aptamer and the affinity polypeptide is the corresponding aptamer ligand.
11. The method of any one of the preceding paragraphs, wherein the Type V CRISPR-Cas effector protein comprises a mutation in a nuclease active site.
12. The method of any one of the preceding paragraphs, wherein the deaminase is a cytosine deaminase and/or an adenine deaminase.
13. The method of any one of the preceding paragraphs, wherein the two or more deaminase fusion proteins comprise the same or different deaminases.
14. The method of paragraph 13, wherein a first deaminase fusion protein of the two or more deaminase fusion proteins comprises a cytosine deaminase and a second deaminase fusion protein of the two or more deaminase fusion proteins comprises an adenine deaminase.
15. The method of any one of paragraphs 12 to 14, wherein the cytosine deaminase is an apolipoprotein B mRNA editing catalytic polypeptide-like (APOBEC), a human activation induced deaminase (hAID), a FERNY deaminase, and/or a CDA1 deaminase, optionally wherein the cytosine deaminase comprises or is the sequence of any one of **SEQ ID NOs:23-32.**
16. The method of paragraph 15, wherein the cytosine deaminase is an APOBEC3A, optionally wherein the APOBEC3A comprises or is the sequence of (e.g., **SEQ ID NO:24**).
17. The method of any one of paragraphs 12 to 16, wherein the adenine deaminase is TadA (tRNA-specific adenosine deaminase) and/or TadA* (evolved tRNA-specific adenosine deaminase), optionally wherein the adenine deaminase comprises or is the sequence of any one of **SEQ ID NOs:33-43.**
18. The method of any one of the preceding paragraphs, further comprising introducing a glycosylase inhibitor, optionally introducing two or more glycosylase inhibitors.
19. The method of paragraph 18, wherein the glycosylase inhibitor is a uracil-DNA glycosylase inhibitor (UGI), optionally wherein the UGI comprises or is the sequence of **SEQ ID NO: 44.**
20. The method paragraph 18 or paragraph 19, wherein the glycosylase inhibitor is recruited to the Type V CRISPR-Cas effector protein and/or the deaminase, optionally via a protein-protein interaction, RNA-protein interaction, or chemical interaction.
21. The method of paragraph 18-20, wherein the glycosylase inhibitor is fused to the Type V CRISPR-Cas effector protein and/or the deaminase.
22. The method of any one of the preceding paragraphs, wherein the Type V CRISPR-Cas effector protein, the Type V CRISPR-Cas fusion protein, the deaminase, and/or the deaminase fusion protein is encoded by a polynucleotide.
23. The method of any one of paragraphs 18 to 22, wherein the glycosylase inhibitor is encoded by a polynucleotide.
24. The method of paragraph 22 or 23, wherein the polynucleotide encoding the Type V CRISPR-Cas effector protein, the polynucleotide encoding the deaminase, and the guide nucleic acid are comprised in one or more expression cassette(s) and/or vector(s).
25. The method of any one of paragraphs 22-24, wherein the polynucleotide encoding the Type V CRISPR-Cas fusion protein, the polynucleotide encoding the deaminase fusion protein, and the guide nucleic acid are comprised in one or more expression cassette(s) and/or vector(s).
26. The method of any one of paragraphs 22-25, wherein the polynucleotide encoding the Type V CRISPR-Cas effector protein, the polynucleotide encoding the deaminase fusion protein, and the recruiting guide nucleic acid are comprised in one or more expression cassette(s) and/or vector(s).
27. The method of any one of paragraphs 23 to 26, wherein the polynucleotide encoding the glycosylase inhibitor is comprised in an expression cassette or vector, optionally in the same expression cassette or vector comprising one or more of the polynucleotide encoding the Type V CRISPR-Cas effector protein, the polynucleotide encoding the deaminase, and the guide nucleic acid, optionally in the same expression cassette or vector comprising one or more of the polynucleotide encoding the Type V CRISPR-Cas fusion protein, the polynucleotide encoding the deaminase fusion protein, and/or the guide nucleic acid, and/or optionally in the same expression cassette or vector comprising one or more of the polynucleotide encoding the Type V CRISPR-Cas effector protein, the polynucleotide encoding the deaminase fusion protein, and/or the recruiting guide nucleic acid.
28. The method of any one of the preceding paragraphs, wherein the Type V CRISPR-Cas effector protein is linked to a polypeptide of interest.
29. The method of paragraph 28, wherein the polypeptide of interest comprises at least one polypeptide having nickase activity, recombinase activity, transposase activity, methylase activity, glycosylase (DNA glycosylase) activity, glycosylase inhibitor activity (e.g., uracil-DNA glycosylase inhibitor (UGI)), demethylase activity, transcription activation activity, transcription repression activity, transcription release factor activity, histone modification activity, nuclease activity, single-strand RNA cleavage activity, double-strand RNA cleavage activity, restriction endonuclease activity (e.g., Fok1), nucleic acid binding activity, methyltransferase activity, DNA repair activity, DNA damage activity, dismutase activity, alkylation activity, depurination activity, oxidation activity, pyrimidine dimer forming activity, integrase activity, transposase activity, polymerase activity, ligase activity, helicase activity, and/or photolyase activity.
30. The method of paragraph 28 or paragraph 29, wherein the polypeptide of interest is encoded by a polynucleotide.
31. The method of any one of the preceding paragraphs, wherein the Type V CRISPR-Cas effector protein is a Cas12a (Cpfl) polypeptide, Cas12b polypeptide, Cas12c (C2c3) polypeptide, Cas12d (CasY) polypeptide, Cas12e (CasX) polypeptide, Cas12g polypeptide, Cas12h polypeptide, Cas12i polypeptide, C2c4 polypeptide, C2c5 polypeptide, C2c8 polypeptide, C2c9 polypeptide, C2c10 polypeptide, Cas14a polypeptide, Cas14b polypeptide, and/or Cas14c polypeptide.
32. The method of any of the preceding paragraphs, wherein the Type V CRISPR-Cas effector protein is a Cas12a (Cpfl) polypeptide, optionally wherein the Cas12a (Cpfl) polypeptide has a sequence of any one of **SEQ ID NOs:3-19** or that is encoded by a polynucleotide having a sequence of any one of **SEQ ID NOs:20-22,** optionally wherein the Cas12a polypeptide is a *Lachnospiraceae* bacterium ND2006 Cas12a (LbCas12a)(LbCpf1) polypeptide (e.g., having a sequence of any one **of SEQ ID NOs:3** or **9-11**), an *Acidaminococcus sp.* Cpfl (AsCas12a) (AsCpfl) polypeptide(e.g., having a sequence **of SEQ ID NO:4**) and/or enAsCas12a polypeptide (e.g., encoded by a polynucleotide having a sequence of any one of **SEQ ID NOs:20-22**).
33. The method of any one of the preceding paragraphs, wherein the target nucleic acid is in an organism, optionally an animal, a plant, a fungus, an archaeon, or a bacterium.
34. The method of paragraph 28, wherein the polynucleotide encoding the Type V CRISPR-Cas effector protein, the polynucleotide encoding the Type V CRISPR-Cas fusion protein, the polynucleotide encoding the deaminase, the polynucleotide encoding the deaminase fusion protein, the polynucleotide encoding the glycosylase inhibitor, and/or the polynucleotide encoding the polypeptide of interest are codon optimized for expression in the organism comprising the target nucleic acid.
35. A nucleic acid construct comprising:
   (a) a Type V Clustered Regularly Interspaced Short Palindromic Repeats (CRISPR)-associated (Cas) (CRISPR-Cas) fusion protein comprising a Type V CRISPR-Cas effector protein fused to a peptide tag;
   (b) a deaminase fusion protein comprising a deaminase fused to an affinity polypeptide that binds to the peptide tag; and
   (c) a guide nucleic acid.
36. A nucleic acid construct comprising:
   (a) a Type V Clustered Regularly Interspaced Short Palindromic Repeats (CRISPR)-associated (Cas) (CRISPR-Cas) effector protein;
   (b) a recruiting guide nucleic acid comprising a guide RNA linked to an RNA recruiting motif; and
   (c) a deaminase fusion protein comprising a deaminase fused to an affinity polypeptide that binds to the RNA recruiting motif.
37. A Type V Clustered Regularly Interspaced Short Palindromic Repeats (CRISPR)-associated (Cas) (CRISPR-Cas) system comprising:
   (a) a Type V CRISPR-Cas fusion protein comprising a Type V CRISPR-Cas effector protein fused to a peptide tag;
   (b) a deaminase fusion protein comprising a deaminase fused to an affinity polypeptide that binds to the peptide tag; and
   (c) a guide nucleic acid comprising a spacer sequence and a repeat sequence, wherein the guide nucleic acid is capable of forming a complex with the Type V CRISPR-Cas effector protein of the Type V CRISPR-Cas fusion protein and the spacer sequence is capable of hybridizing to a target nucleic acid, thereby guiding the Type V CRISPR-Cas fusion protein to the target nucleic acid, and wherein the deaminase fusion protein is recruited to the Type V CRISPR-Cas fusion protein and target nucleic acid by the binding of the affinity polypeptide to the peptide tag that is fused to the Type V CRISPR-Cas fusion protein, whereby the system is capable of modifying (e.g., cleaving or editing) the target nucleic acid.
38. A Type V Clustered Regularly Interspaced Short Palindromic Repeats (CRISPR)-associated (Cas) (CRISPR-Cas) system comprising:
   (a) a Type V CRISPR-Cas effector protein;
   (b) a recruiting guide nucleic acid comprising a guide RNA linked to an RNA recruiting motif, and
   (c) a deaminase fusion protein comprising a deaminase fused to an affinity polypeptide that binds to the RNA recruiting motif, wherein the recruiting guide nucleic acid comprises a spacer sequence and a repeat sequence, wherein the recruiting guide nucleic acid is capable of forming a complex with the Type V CRISPR-Cas effector protein and the guide RNA is capable of hybridizing to a target nucleic acid, thereby guiding the Type V CRISPR-Cas effector protein to the target nucleic acid, and wherein the deaminase fusion protein is recruited to the Type V CRISPR-Cas effector protein and target nucleic acid by the binding of the affinity polypeptide to the RNA recruiting motif that is fused to the recruiting guide nucleic acid, whereby the system is capable of modifying (e.g., cleaving or editing) the target nucleic acid.
39. The nucleic construct of paragraph 35 or paragraph 36, or the CRISPR-Cas system of paragraph 37 or paragraph 38, wherein the Type V CRISPR-Cas effector protein is a Cas12a (Cpfl) polypeptide, Cas12b polypeptide, Cas12c (C2c3) polypeptide, Cas12d (CasY) polypeptide, Cas12e (CasX) polypeptide, Cas12g polypeptide, Cas12h polypeptide, Cas12i polypeptide, C2c4 polypeptide, C2c5 polypeptide, C2c8 polypeptide, C2c9 polypeptide, C2c10 polypeptide, Cas14a polypeptide, Cas14b polypeptide, and/or Cas14c polypeptide.
40. The nucleic acid construct of paragraph 35 or paragraph 36, or the CRISPR-Cas system of paragraph 37 or paragraph 38, wherein the Type V CRISPR-Cas effector protein is a Cas12a (Cpfl) polypeptide, optionally wherein the Cas12a (Cpfl) polypeptide has a sequence of any one of **SEQ ID NOs:3-19** or that is encoded by a polynucleotide having a sequence of any one of **SEQ ID NOs:20-22,** optionally wherein the Cas12a polypeptide is a *Lachnospiraceae* bacterium ND2006 Cas12a (LbCas12a)(LbCpf1) polypeptide (e.g., having a sequence of any one **of SEQ ID NOs:3** or **9-11**), an *Acidaminococcus sp.* Cpfl (AsCas12a) (AsCpfl) polypeptide(e.g., having a sequence **of SEQ ID NO:4**) and/or enAsCas12a polypeptide (e.g., encoded by a polynucleotide having a sequence of any one of **SEQ ID NOs:20-22**).
41. An expression cassette comprising the nucleic acid construct of any one of paragraphs 35, 36, 39, or 40.
42. An expression cassette comprising the CRISPR-Cas system of any one of paragraphs 37 to 40.
43. A cell comprising the nucleic acid construct of any one of paragraphs 35, 36, 39, or 40, or the expression cassette of paragraph 41.
44. A cell comprising the CRISPR-Cas system of any one of paragraphs 37 to 40 or the expression cassette of paragraph 42.

## Claims

1. A method of modifying a target nucleic acid, the method comprising:
contacting the target nucleic acid with:
(a) a Type V Clustered Regularly Interspaced Short Palindromic Repeats (CRISPR)-associated (Cas) (CRISPR-Cas) effector protein;
(b) a deaminase, optionally wherein the target nucleic acid is contacted with two or more deaminases; and
(c) a guide nucleic acid, wherein the deaminase is recruited to the Type V CRISPR-Cas effector protein thereby modifying the target nucleic acid, optionally wherein the Type V CRISPR-Cas effector protein, the deaminase and guide nucleic acid are co-expressed.

2. The method of claim 1 wherein the Type V Clustered Regularly Interspaced Short Palindromic Repeats (CRISPR)-associated (Cas) (CRISPR-Cas) effector protein is:
i) fused to a peptide tag; and the deaminase is a fusion protein which comprises a deaminase fused to an affinity polypeptide that binds to the peptide tag; or
ii) fused to an affinity polypeptide that binds to a peptide tag, and wherein the deaminase is a deaminase fusion protein which comprises a deaminase fused to the peptide tag.

3. The method of claim 1, wherein:
the guide nucleic acid is a recruiting guide nucleic acid comprising a guide RNA linked to an RNA recruiting motif, and the deaminase is a deaminase fusion protein comprising a deaminase fused to an affinity polypeptide that binds to the RNA recruiting motif; and
optionally wherein the Type V CRISPR-Cas effector protein, the deaminase fusion protein and the recruiting guide nucleic acid are co-expressed, thereby modifying the target nucleic acid.

4. The method of claim 2, wherein:
i) the peptide tag comprises 2 or more copies of the peptide tag;
ii) the peptide tag is a GCN4 peptide repeat unit, a c-Myc affinity tag, an HA affinity tag, a His affinity tag, an S affinity tag, a methionine-His affinity tag, an RGD-His affinity tag, a FLAG octapeptide, a strep tag or strep tag II, a V5 tag, and/or a VSV-G epitope;
iii) the affinity polypeptide is an antibody, optionally wherein the antibody is a scFv antibody; or
iv) any combination of i) to iii) above.

5. The method of claim 3, wherein:
i) the recruiting guide nucleic acid is linked to two or more RNA recruiting motifs, optionally wherein the two or more RNA recruiting motifs are the same RNA recruiting motif or different RNA recruiting motifs; and/or
ii) the RNA recruiting motif is a telomerase Ku binding motif (e.g., Ku binding hairpin) and the affinity polypeptide is a Ku polypeptide (e.g., Ku heterodimer); the RNA recruiting motif is a telomerase Sm7 binding motif and the affinity polypeptide is a Sm7 polypeptide; the RNA recruiting motif is an MS2 phage operator stem-loop and the affinity polypeptide is a MS2 Coat Protein (MCP); the RNA recruiting motif is a PP7 phage operator stem-loop and the affinity polypeptide is a PP7 Coat Protein (PCP); the RNA recruiting motif is an SfMu phage Com stem-loop and the affinity polypeptide is a Com RNA binding protein; the RNA recruiting motif is a Pumilio/fem-3 mRNA binding factor (PUF) and the affinity polypeptide is a PUF binding site (PBS) polypeptide; and/or the RNA recruiting motif is a synthetic RNA-aptamer and the affinity polypeptide is the corresponding aptamer ligand.

6. The method of any one of the preceding claims, wherein:
i) the Type V CRISPR-Cas effector protein comprises a mutation in a nuclease active site;
ii) the deaminase is a cytosine deaminase and/or an adenine deaminase;
iii) the two or more deaminase fusion proteins comprise the same or different deaminases, optionally, wherein a first deaminase fusion protein of the two or more deaminase fusion proteins comprises a cytosine deaminase and a second deaminase fusion protein of the two or more deaminase fusion proteins comprises an adenine deaminase;
iv) the deaminase is a cytosine deaminase and wherein the cytosine deaminase is:
a. an apolipoprotein B mRNA editing catalytic polypeptide-like (APOBEC), a human activation induced deaminase (hAID), a FERNY deaminase, and/or a CDA1 deaminase, optionally wherein the cytosine deaminase comprises or is the sequence of any one of **SEQ ID NOs:23-32;** or
b. wherein the cytosine deaminase is an APOBEC3A, optionally wherein the APOBEC3A comprises or is the sequence of (e.g., **SEQ ID NO:24**);
v) the deaminase is an adenine deaminase and wherein the adenine deaminase is TadA (tRNA-specific adenosine deaminase) and/or TadA* (evolved tRNA-specific adenosine deaminase), optionally wherein the adenine deaminase comprises or is the sequence of any one of **SEQ ID NOs:33-43;** or
vi) any combination of i) to v) above.

7. The method of any one of the preceding claims, further comprising introducing a glycosylase inhibitor, optionally:
i) introducing two or more glycosylase inhibitors;
ii) wherein the glycosylase inhibitor is a uracil-DNA glycosylase inhibitor (UGI), optionally wherein the UGI comprises or is the sequence of **SEQ ID NO:44;**
iii) wherein the glycosylase inhibitor is recruited to the Type V CRISPR-Cas effector protein and/or the deaminase, optionally via a protein-protein interaction, RNA-protein interaction, or chemical interaction;
iv) wherein the glycosylase inhibitor is fused to the Type V CRISPR-Cas effector protein and/or the deaminase;
v) wherein the glycosylase inhibitor is encoded by a polynucleotide; or
vi) any combination of i) to v) above.

8. The method of any one of the preceding claims, wherein the Type V CRISPR-Cas effector protein, the Type V CRISPR-Cas fusion protein, the deaminase, and/or the deaminase fusion protein is encoded by a polynucleotide, optionally:
i) wherein the polynucleotide encoding the Type V CRISPR-Cas effector protein, the polynucleotide encoding the deaminase, and the guide nucleic acid are comprised in one or more expression cassette(s) and/or vector(s);
ii) wherein the polynucleotide encoding the Type V CRISPR-Cas fusion protein, the polynucleotide encoding the deaminase fusion protein, and the guide nucleic acid are comprised in one or more expression cassette(s) and/or vector(s);
iii) wherein the polynucleotide encoding the Type V CRISPR-Cas effector protein, the polynucleotide encoding the deaminase fusion protein, and the recruiting guide nucleic acid are comprised in one or more expression cassette(s) and/or vector(s) and/or
iv) wherein the polynucleotide encoding the glycosylase inhibitor is comprised in an expression cassette or vector, optionally in the same expression cassette or vector comprising one or more of the polynucleotide encoding the Type V CRISPR-Cas effector protein, the polynucleotide encoding the deaminase, and the guide nucleic acid, optionally in the same expression cassette or vector comprising one or more of the polynucleotide encoding the Type V CRISPR-Cas fusion protein, the polynucleotide encoding the deaminase fusion protein, and/or the guide nucleic acid, and/or optionally in the same expression cassette or vector comprising one or more of the polynucleotide encoding the Type V CRISPR-Cas effector protein, the polynucleotide encoding the deaminase fusion protein, and/or the recruiting guide nucleic acid.

9. The method of any one of the preceding claims, wherein the Type V CRISPR-Cas effector protein is linked to a polypeptide of interest, optionally:
i) wherein the polypeptide of interest comprises at least one polypeptide having nickase activity, recombinase activity, transposase activity, methylase activity, glycosylase activity, glycosylase inhibitor activity, demethylase activity, transcription activation activity, transcription repression activity, transcription release factor activity, histone modification activity, nuclease activity, single-strand RNA cleavage activity, double-strand RNA cleavage activity, restriction endonuclease activity, nucleic acid binding activity, methyltransferase activity, DNA repair activity, DNA damage activity, dismutase activity, alkylation activity, depurination activity, oxidation activity, pyrimidine dimer forming activity, integrase activity, transposase activity, polymerase activity, ligase activity, helicase activity, and/or photolyase activity;
ii) wherein the polypeptide of interest is encoded by a polynucleotide; and/or
iii) wherein the polynucleotide encoding the Type V CRISPR-Cas effector protein, the polynucleotide encoding the Type V CRISPR-Cas fusion protein, the polynucleotide encoding the deaminase, the polynucleotide encoding the deaminase fusion protein, the polynucleotide encoding the glycosylase inhibitor, and/or the polynucleotide encoding the polypeptide of interest are codon optimized for expression in the organism comprising the target nucleic acid.

10. The method of any one of the preceding claims, wherein the Type V CRISPR-Cas effector protein is:
i) a Cas12a (Cpfl) polypeptide, Cas12b polypeptide, Cas12c (C2c3) polypeptide, Cas12d (CasY) polypeptide, Cas12e (CasX) polypeptide, Cas12g polypeptide, Cas12h polypeptide, Cas12i polypeptide, C2c4 polypeptide, C2c5 polypeptide, C2c8 polypeptide, C2c9 polypeptide, C2c10 polypeptide, Cas14a polypeptide, Cas14b polypeptide, and/or Cas14c polypeptide; and/or
ii) a Cas12a (Cpfl) polypeptide, optionally wherein the Cas12a (Cpfl) polypeptide has a sequence of any one **of SEQ ID NOs:3-19** or that is encoded by a polynucleotide having a sequence of any one **of SEQ ID NOs:20-22,** optionally wherein the Cas12a polypeptide is *a Lachnospiraceae* bacterium ND2006 Cas12a (LbCas12a)(LbCpf1) polypeptide, an *Acidaminococcus sp.* Cpfl (AsCas12a) (AsCpfl) polypeptide and/or enAsCas12a polypeptide;
optionally:
wherein the target nucleic acid is in an organism, optionally an animal, a plant, a fungus, an archaeon, or a bacterium.

11. A nucleic acid construct comprising:
(i) a Type V Clustered Regularly Interspaced Short Palindromic Repeats (CRISPR)-associated (Cas) (CRISPR-Cas) fusion protein comprising a Type V CRISPR-Cas effector protein fused to a peptide tag; a deaminase fusion protein comprising a deaminase fused to an affinity polypeptide that binds to the peptide tag; and a guide nucleic acid; or
(ii) a Type V Clustered Regularly Interspaced Short Palindromic Repeats (CRISPR)-associated (Cas) (CRISPR-Cas) effector protein; a recruiting guide nucleic acid comprising a guide RNA linked to an RNA recruiting motif; and a deaminase fusion protein comprising a deaminase fused to an affinity polypeptide that binds to the RNA recruiting motif.

12. A Type V Clustered Regularly Interspaced Short Palindromic Repeats (CRISPR)-associated (Cas) (CRISPR-Cas) system comprising:
(i) a Type V CRISPR-Cas fusion protein comprising a Type V CRISPR-Cas effector protein fused to a peptide tag; a deaminase fusion protein comprising a deaminase fused to an affinity polypeptide that binds to the peptide tag; and a guide nucleic acid comprising a spacer sequence and a repeat sequence, wherein the guide nucleic acid is capable of forming a complex with the Type V CRISPR-Cas effector protein of the Type V CRISPR-Cas fusion protein and the spacer sequence is capable of hybridizing to a target nucleic acid, thereby guiding the Type V CRISPR-Cas fusion protein to the target nucleic acid, and wherein the deaminase fusion protein is recruited to the Type V CRISPR-Cas fusion protein and target nucleic acid by the binding of the affinity polypeptide to the peptide tag that is fused to the Type V CRISPR-Cas fusion protein, whereby the system is capable of modifying the target nucleic acid; or
(ii) a Type V CRISPR-Cas effector protein; a recruiting guide nucleic acid comprising a guide RNA linked to an RNA recruiting motif, and a deaminase fusion protein comprising a deaminase fused to an affinity polypeptide that binds to the RNA recruiting motif, wherein the recruiting guide nucleic acid comprises a spacer sequence and a repeat sequence, wherein the recruiting guide nucleic acid is capable of forming a complex with the Type V CRISPR-Cas effector protein and the guide RNA is capable of hybridizing to a target nucleic acid, thereby guiding the Type V CRISPR-Cas effector protein to the target nucleic acid, and wherein the deaminase fusion protein is recruited to the Type V CRISPR-Cas effector protein and target nucleic acid by the binding of the affinity polypeptide to the RNA recruiting motif that is fused to the recruiting guide nucleic acid, whereby the system is capable of modifying the target nucleic acid.

13. The nucleic construct of claim 11 or the CRISPR-Cas system of claim 12, wherein the Type V CRISPR-Cas effector protein is a Cas12a (Cpfl) polypeptide, Cas12b polypeptide, Cas12c (C2c3) polypeptide, Cas12d (CasY) polypeptide, Cas12e (CasX) polypeptide, Cas12g polypeptide, Cas12h polypeptide, Cas12i polypeptide, C2c4 polypeptide, C2c5 polypeptide, C2c8 polypeptide, C2c9 polypeptide, C2c10 polypeptide, Cas14a polypeptide, Cas14b polypeptide, and/or Cas14c polypeptide , optionally wherein the Type V CRISPR-Cas effector protein is a Cas12a (Cpfl) polypeptide, optionally wherein the Cas12a (Cpfl) polypeptide has a sequence of any one of **SEQ ID NOs:3-19** or that is encoded by a polynucleotide having a sequence of any one of **SEQ ID NOs:20-22,** optionally wherein the Cas12a polypeptide is *a Lachnospiraceae* bacterium ND2006 Cas12a (LbCas12a)(LbCpf1) polypeptide, an *Acidaminococcus sp.* Cpfl (AsCas12a) (AsCpfl) polypeptide(and/or enAsCas12a polypeptide.

14. An expression cassette comprising:
i) the nucleic acid construct of claim 11 or claim 13; or
ii) the CRISPR-Cas system of claim 12 or claim 13.

15. A cell comprising:
i) the nucleic acid construct of claim 11 or claim 13;
ii) the expression cassette of claim 14;
ii) the CRISPR-Cas system of claim 12 or claim 13.
